(19) Europäisches Patentamt
European Patent Office
Office européen des brevets

(11) Publication number: **0 248 597 A2**

# (12) EUROPEAN PATENT APPLICATION

(21) Application number: **87304723.7**

(22) Date of filing: **28.05.87**

(51) Int. Cl.[4]: **C07C 149/36**, C07C 149/24, C07C 149/247, C07C 147/02, C07C 147/05, C07C 147/06, C07K 5/06, C07K 5/08

(30) Priority: **29.05.86 US 867995**

(43) Date of publication of application: **09.12.87 Bulletin 87/50**

(84) Designated Contracting States: **AT BE CH DE ES FR GB GR IT LI LU NL SE**

(71) Applicant: **ORTHO PHARMACEUTICAL CORPORATION**
**U.S. Route 202 P.O. Box 300**
**Raritan New Jersey 08869-0602(US)**

(72) Inventor: **Wachter, Michael Paul**
**52 North Street**
**P.O. Box 362 Bloomsbury NJ 08804(US)**
Inventor: **Ferro, Michael Paul**
**161 Woods Road**
**Somerville, NJ 08876(US)**

(74) Representative: **Jones, Alan John et al**
**CARPMAELS & RANSFORD 43 Bloomsbury Square**
**London, WC1A 2RA(GB)**

## (54) Phospholipase A2 inhibitors and methods of synthesis.

(57) Compounds that inhibit the hydrolytic activity of phospholipase $A_2$ are described which are pharmacologically active in the treatment of inflammation and allergic conditions including asthma. Methods for synthesizing and using those compounds are described.

EP 0 248 597 A2

# PHOSPHOLIPASE $A_2$ INHIBITORS AND METHODS OF SYNTHESIS

## Technical Field

The present invention relates to novel compounds that inhibit the hydrolytic activity of phospolipase $A_2$ and to methods for synthesizing those compounds. The compounds are pharmacologically active in the treatment of inflammation and allergic conditions including asthma.

## Background

Leukotrienes and prostaglandins have both been detected in the fluids and exudates of inflammatory reactions and in anaphylactic reactions in the lungs. Like leukotrienes, prostaglandins are potent vasodilators and are important mediators of various aspects of the inflammatory response.

Arachidonic acid, the starting material of leukotriene and prostaglandin biosynthesis, is released from cell membrane phospholipids through the activation of cellular phospholipases and, in particular, phospholipase $A_2$ (also referred to as "$PA_2$").

As a consequence, pharmacologically active compounds that inhibit the hydrolytic activity of phospolipase $A_2$ can provide more complete relief from inflammatory and allergic conditions than currently available anti-inflammatory compounds by inhibiting the production of both leukotrienes and prostaglandins.

Leukotrienes are peptidolipids that are derived from arachidonic acid and are recognized as substances that cause smooth muscle, in particular, bronchial smooth muscle, to contract slowly (formerly referred to as "slow reacting substances"). In contrast to the rapid (within seconds) histamine-induced contraction of smooth muscle, leukotrienes require several minutes to produce maximum contraction.

SRS-A (slow reacting substance of anaphylaxis) is one leukotriene that is involved in inflammation and allergies including asthma. It was first isolated from the immunologically challenged lungs of guinea pigs sensitized to ovalbumin. [Morris et al., Nature, 285, 104 (1980).] Leukotriene $D_4$ ($LTD_4$) and the related leukotrienes $LTC_4$ and $LTE_4$ are other potent contractile agents that act on bronchial smooth muscle and cause vasoconstriction, bronchospasm and the increased vascular permeability associated with asthma and other immediate hypersensitivity diseases.

SRS-A is released from the cells of mammals during an inflammatory condition or an allergic reaction. The SRS-A excreted by the cells, like histamine, contracts smooth muscle tissue producing inflammatory and allergic responses including asthmatic attacks. It has been suggested that histamine and SRS-A act synergistically on smooth muscle and blood vessels, and that both agents contribute to the secondary release of prostaglandins.

Leukotrienes are prepared in vivo following a series of reactions that are intitiated by the conversion of arachidonic acid by fatty acid lipoxygenases into hydroperoxy derivatives including HPETE (hydroperoxyeicosatetraenoic acid) which may be converted to HETE (hydroxyeicosatetraenoic acid). In a similar manner, prostaglandins are prepared in vivo after a series of reactions that are initiated by the conversion of arachidonic acid by fatty acid cyclooxygenases into prostaglandin endoperoxides.

Currently available anti-inflammatory compounds include the corticosteroids and the nonsteroidal anti-inflammatory drugs. The corticosteroids often produce undesirable side effects. The non-steroidal drugs, which include indomethacin, naproxen, ibuprofen, tolectin and fenoprofen, inhibit prostaglandin synthesis but do not inhibit leukotriene synthesis.

Thus, compounds that inhibit the hydrolytic activity of phospholipase $A_2$ to decrease the production of arachidonic acid and, as a result, the production of leukotrienes and prostaglandins would be beneficial in the treatment of inflammation and allergies. Such compounds would also be useful in the treatment of conditions including arthritis, psoriasis and asthma.

Few compounds have been described in the literature as being effective cellular phospholipase $A_2$ inhibitors.

The following chemically related compounds have been reported. Gleason et al. * disclosed the synthesis of 2-Nor-$LTD_1$ analogs, including 4R-hydroxy-5S-cysteinylglycyl-6Z-nonadecenoic acid, which are

reported to be antagonists of $LTD_4$ on the smooth muscle tissue of the trachea in guinea pigs.

Perchonock et al. * reported on a series of desamino-2-Nor-leukotriene analogs that antagonize the $LTD_4$-induced contraction of isolated guinea pig trachea. The most potent analog was reported to be 4-Hydroxy-5-(2-carboxyethylthio)-6Z-nonadecenoic acid.

The synthesis of structural analogs of 4R-hydroxy-5S-cysteinylglycyl-6Z-nonadecenoic acid and the antagonistic activity of such analogs on $LTD_4$-induced contractive responses on isolated guinea pig trachea was also reported. *

5S-Hydroxy-6R-Benzylthio leukotriene derivatives were reported as inhibitors of leukotriene synthesis and as inhibitors of 5-lipoxygenase activity. Japanese application No. J57118555.

Sulfur-containing 5-hydroxy-alkanoic acid derivatives are said to be useful as SRS-A antagonists in the treatment of allergic conditions including asthma (U.S. Patent No. 4,513,005).

Chan et al., U.S. Patent Nos. 4,345,084 and 4,500,462, disclose unsaturated eicosanoic acids and derivatives thereof that reportedly inhibit the synthesis of SRS-A and are useful as anti-allergy, anti-asthma and anti-inflammatory agents.

## Brief Summary of the Invention

The present invention relates to novel compounds, their use and methods for synthesizing such compounds. The compounds of the present invention inhibit the hydrolytic activity of phospholipase $A_2$ and are pharmacologically active in reducing inflammation and in controlling allergic responses.

In particular, the invention relates to compounds having structures that correspond to the formula:

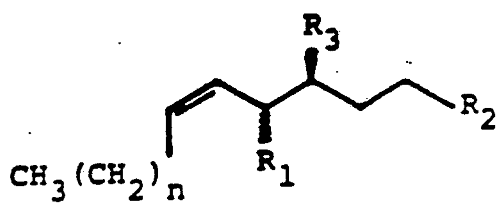

wherein $R_1$ is selected from the group consisting of unsubstituted or substituted thiophenyl or phenylsulfonyl, cysteinyl, glutathionyl, cysteinylglycinyl, unsubstituted or substituted lower thioalkylamino or sulfonylalkylacetamido, and unsubstituted or substituted lower thioalkylacetamido or sulfonylalkylacetamido radicals;

$R_2$ is selected form the group consisting of lower alkyl hydroxy, lower alkyl tetrahydropyranyloxy, lower alkyl carboxy and lower alkenyl radicals:

$R_3$ is selected from the group consisting of hydroxy, carbonyl, oximino and lower alkyl radicals; and

n is an integer from about 10 to about 14.

Also contemplated are the pharmaceutically acceptable salts, esters, epoxides and lactones that correspond to the foregoing formula. In particular, $R_1$ and $R_3$ taken together can form an epoxide, and $R_2$ and $R_3$ taken together may form a lactone.

The compounds corresponding to the above formula, have been found to be effective at inhibiting the hydrolytic activity of phospholipase $A_2$.

Preferred compounds of this invention have structures that correspond to the above formula wherein $R_1$ is substituted lower thioalkylacetamido, $R_2$ is lower alkyl hydroxy, $R_3$ is hydroxy and n is 11; wherein $R_1$ is glutathionyl, $R_2$ is lower alkyl tetrahydropyranyloxy, $R_3$ is hydroxy and n is 11; wherein $R_1$ is cysteinyl, $R_2$ is lower alkyl hydroxy, $R_3$ is hydroxy and n is 11; and wherein $R_1$ is substituted lower thioalkylacetamido, $R_2$ is lower alkyl hydroxy, $R_3$ is oximino and n is 11.

*Paper entitled "2-Nor-Leukotrienes: A Novel Series of Leukotriene Antagonists", presented at the 186th National Meeting of the American Chemical Society-Division of Medicinal Chemistry, September 1983.

*Paper entitled "Synthesis and $LTD_4$-Antagonist Activity of Desamino-2-Nor-Leukotriene Analogs", presented at the 188th National Meeting of the American Chemical Society-Division of Medicinal Chemistry, August 1984.

As further described herein, the foregoing preferred compounds are methyl N-acetyl-S-6-[5S, 6R)-1,5-dihydroxy-7(Z)-eicosenyl]-(R)-cysteinate; S-6-[(5S,6R)-5-hydroxy-1-(2-tetrahydropyranyloxy)-7(Z)-eicos enyl] glutathione triethylammonium salt dihydrate; (5S, 6R)-6-[S-(R)-cysteinyl]-7(Z)-eicosen-1,5-diol hemihydrate; and methyl N-acetyl-S-6-[(5S, 6R)-1-hydroxy-5-oximino-7(Z)-eicosenyl)]-(R)-cysteinate, respectively.

The present invention also contemplates a pharmaceutical composition that comprises an effective amount of a compound (as described above) dispersed in a physiologically acceptable carrier. In preferred practice, the pharmaceutical composition containing the compound is capable of inhibiting the hydrolytic activity of phospholipase $A_2$ and is effective in the treatment of inflammatory condition and allergic responses without the side effects normally associated with steroids.

Further contemplated are methods for reducing inflammation and controlling allergic responses in mammals. The methods comprise administering to the mammal a pharmaceutical composition that includes, as the active ingredient, an effective amount of a compound (as described above) in a physiologically acceptable carrier. Oral administration of the composition is preferred.

As will be further disclosed herein, methods for synthesizing the compounds are also contemplated.

The present invention provides several benefits and advantages.

A particular benefit of the invention is the preparation of pharmacologically active compounds that are useful in treating inflammatory conditions and allergic conditions.

A particular advantage of one method of the present invention is the synthesis of compounds of high purity in relatively high yields.

Another benefit of the present invention is that the pharmacologically active compounds described herein are potent inhibitors of $PA_2$ and thus provide a further means for studying that biological process.

Still further benefits and advantages of the present invention will be apparent to those skilled in the art from the detailed description and Examples that follow.

## Detailed Description of the Invention

Novel compounds, pharmaceutical compositions containing such compounds as an active ingredient, methods of treating a mammal exhibiting an inflammatory or allergic condition and methods for synthesizing the compounds are contemplated.

In each compound of the present invention having a structure that corresponds to the foregoing formula, when $R_1$ is a sulfur-containing radical, the sulfur atom is bonded to the compound (see Table 1 herein).

Exemplary compounds of the present invention whose structures conform to the formula above are listed in Table 1 below.

TABLE 1

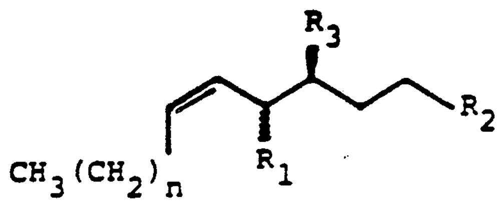

| Product of Example No. | $R_1$ | $R_2$ | $R_3$ | n |
|---|---|---|---|---|
| 1(e) | ($R_1$-$R_3$ epoxide) | $-CH_2CH_2O(C_5H_9O)$* | --- | 11 |
| 2 | $-SCH_2CH(COOCH_3)NHCOCH_3$ | $-CH_2CH_2OH$ | -OH | 11 |
| 3 | -Glutathionyl | $-CH_2CH_2O(C_5H_9O)$ | -OH | 11 |
| 4 | -Cysteinyl | $-CH_2CH_2OH$ | -OH | 11 |
| 5 | $-SCH_2CH_2NHCOCH_3$ | $-CH_2CH_2OH$ | -OH | 11 |
| 6 | $-SCH_2CH(COOCH_3)NHCOCH_3$ | $-CH_2CH_2O(C_5H_9O)$ | -OH | 11 |
| 7 | $-SCH_2CH(COOCH_3)NH_2$ | $-CH_2CH_2OH$ | -OH | 11 |
| 8 | $-SC_6H_5$** | $-CH_2CH_2O(C_5H_9O)$ | -OH | 11 |
| 9 | -*para*-$SC_6H_4Cl$*** | $-CH_2CH_2O(C_5H_9O)$ | -OH | 11 |
| 10 | $-SO_2CH_2CH(COOCH_3)NHCOCH_3$ | $-CH_2CH_2OH$ | -OH | 11 |
| 11 | $-SC_6H_5$ | $-CH_2CH_2OH$ | -OH | 11 |
| 12 | -*para*-$SC_6H_4Cl$ | $-CH_2CH_2OH$ | -OH | 11 |
| 13 | $-SCH_2CH(COOCH_3)NHCOCH_3$ | $-CH_2CH_2O(C_5H_9O)$ | =O | 11 |
| 14 | $-SCH_2CH(COOCH_3)NHCOCH_3$ | $-CH_2CH_2OH$ | =NOH | 11 |
| 15 | $-SCH_2CH(COOCH_3)NHCOCH_3$ | $-CH_2CH_2(C_5H_9O)$ | -OH | 13 |
| 16 | $-SCH_2CH(COOCH_3)NHCOCH_3$ | $-CH_2CH_2OH$ | -OH | 13 |
| 17 | ($R_1$-$R_3$ epoxide) | $-CH{=}C(CH_3)_2$ | --- | 11 |
| 18 | $-SC_6H_5$ | $-CH{=}C(CH_3)_2$ | $-(OH)CH_3$ | 11 |
| 19 | $-SC_6H_5$ | ($R_2$-$R_3$ lactone) | --- | 11 |
| 20 | $-SC_6H_5$ | $-CH_2COO^-Na^+$ | -OH | 11 |

* $(C_5H_9O)$ = tetrahydropyranyl.
** $C_6H_5$ = phenyl.
*** $C_6H_4Cl$ = *para*-chloro phenyl.

The term "phenyl", as used herein alone or in combination with other terms as in "thiophenyl" and "phenylsulfonyl" indicates aromatic hydrocarbon groups which can be unsubstituted or substituted with one or more lower alkyl radicals or halogens selected from chloro, bromo, iodo and fluoro functionalities.

As used herein, the term "lower alkyl", in its various uses, indicates a branched or straight chain hydrocarbon having from 1 to about 8 carbon atoms. Lower alkyl radicals include, for example, methyl, ethyl, propyl, isopropyl, n-butyl, sec-butyl, t-butyl, n-pentyl, 2-methyl-3-butyl, 1-methylbutyl, 2-methylbutyl, neopentyl, n-hexyl, 1-methylpentyl, 3-methylpentyl, 1-ethylbutyl, 2-ethylbutyl, 2-hexyl, 3-hexyl, 1-octyl, 2-octyl and the like.

Throughout this application, the term "lower" refers to "alkyl", unless stated otherwise. Thus, the term "lower thioalkylamino", for example, indicates a lower alkyl moiety having both thiol and amine functionalities.

In a like manner, the term "lower alkenyl" refers to a branched or straight chain unsaturated hydrocarbon having from 1 to about 8 carbon atoms. Suitable lower alkenyl radicals include foregoing alkyl radicals having at least one carbon-carbon double bond, preferably a single carbon-carbon double bond, therein.

The term "substituted" used with reference to thiophenyl or thioalkylamino, for example, indicates a phenyl or alkyl group, respectively, that includes a halogen selected from chloro, bromo, iodo, and fluoro functionalities or a -COOR group wherein R is lower alkyl, but preferably where R is methyl.

A pharmaceutical composition that comprises an anti-inflammatory amount of an above-described compound dispersed in a pharmaceutically acceptable carrier is also contemplated herein. The composition comprises a unit dosage of the compound.

The compounds of this invention are capable of inhibiting the hydrolytic activity of phospholipase $A_2$. In preferred practice, the compound is capable of inhibiting the hydrolytic activity of $PA_2$ in the amount at which that compound is present in the pharmaceutical composition, when that composition is introduced as a unit dose into a appropriate mammal.

The term "unit dosage" and its grammatical equivalents are used herein to refer to physically discrete units suitable for administration to human patients and to warm-blooded mammals. Each unit contains a predetermined effective, anti-inflammatory or anti-allergic amount of the active ingredient calculated to produce the desired anti-inflammatory or anti-allergic effect in association with the required physiologically tolerable carrier, e.g., a diluent or a vehicle.

The specification for the novel unit dosage forms of this invention are dictated by and are directly dependent on (a) the unique characteristics of the active ingredient, and (b) the limitations inherent in the art of compounding such an active ingredient for therapeutic use in humans and other mammals. Examples of suitable unit dosage forms in accord with this invention are tablets, capsules, pills, powder packets, granules, wafers and the like segregated multiples of any of the foregoing, along with liquid solution, liquid suspensions, elixirs and aerosol suspensions.

The active ingredient is referred to herein as being dispersed in the carrier. Thus, the dispersion formed can be a simple admixture, a non-settling dispersion as in the case of certain emulsions or as an ultimate dispersion, a true solution. In such compositions, the active ingredient is ordinarily present in an amount of at least about 0.5 percent by weight based on the total weight of the composition to about 90 percent by weight.

The amount of active ingredient that is administered in vivo depends on the age and weight of the mammal treated, the particular inflammatory or allergic condition to be treated, the frequency of administration, and the route of administration. The dose range can be about 0.001 to about 50 milligrams per kilogram of body weight, more preferably about 0.01 to about 10 milligrams per kilogram of body weight per day and most preferably about 0.1 to about 5 miligrams per kilogram of body weight per day. The human adult dose is in the range of about 100 to about 500 milligrams daily, given as a single dose or in 3 or 4 divided doses. Veterinary dosages correspond to human dosages with the amounts administered being in proportion to the weight of the animal as compared to adult humans.

Orally administered unit doses containing about 1 to about 50 milligrams of active compound per kilogram of laboratory rat body weight (e.g., about 200 grams each) are useful in reducing inflammation and in controlling allergic responses.

However, it will be understood that the amount administered is determined by the physician or veterinarian in light of the relevant circumstances including the condition to be treated, the compound to be administered and the route of administration. Therefore, the foregoing dosage ranges are not intended to limit the scope of this invention in any way.

Physiologically tolerable carriers are well known in the art. Suitable liquid carriers include aqueous solutions that contain no materials in addition to the compound, or that contain a buffer such as sodium phosphate at a physiological pH value, saline and the like.

Liquid compositions can also contain liquid phases in addition to or to the exclusion of water. Exemplary of such additional liquid phases are glycerin and vegetable oils including peanut oil and cottonseed oil.

Suitable solid carriers (diluents) include those materials usually used in the manufacture of pills or tablets, and include corn starch, lactose, dicalcium phosphate, thickeners such as tragacanth and methylcellulose U.S.P., finely divided silica, polyvinylpyrrolidone, magnesium stearate and the like. Antioxidants such as methylparaben and propylparaben can be present in both solid and liquid compositions, as can sweeteners such as cane sugar, beet sugar, sodium saccharin, sodium cyclamate and the dipeptide methyl ester sweetener sold under the trademark NUTRASWEET (aspartame) by G. D. Searle Co., Skokie, IL.

Methods for reducing an inflammatory condition and for controlling an allergic condition in a mammal exhibiting an inflammatory or allergic condition are also contemplated. The methods comprise administering to that mammal an effective amount of a pharmaceutical composition that includes a unit dose of an active ingredient that is the aforementioned compound dispersed in a pharmaceutically acceptable carrier. The pharmaceutical composition is preferably maintained within the mammal until the compound is cleared from the body of the mammal by natural means such as metabolism or excretion.

The pharmaceutical composition can be administered orally, by injection, by inhalation (for example, in the form of an aerosol, micropulverized powder or nebulized solution) topically or by any other means well known in the art. In preferred practice, the composition is administered as an aerosol, orally as a tablet, capsule or aqueous dispersion, or topically in the form of a lotion, cream or ointment.

When administered as an aerosol, the compound is dissolved in a suitable pharmaceutically acceptable solvent (for example, ethyl alcohol, water or combinations of miscible, physiologically acceptable solvents, as are well known) and mixed with a pharmaceutically acceptable propellant. Such aerosol compositions are packaged for use in a pressurized container fitted with an aerosol valve suitable for release of the pressurized composition. Preferably, the aerosol valve is a metered valve which on activation releases a predetermined effective dose of the aerosol composition.

Inasmuch as a pharmaceutical composition can be administered 3 or more times daily (during a 24 hour period), the method of alleviating inflammation or controlling allergic responses can include the serial administration of the pharmaceutical composition into the treated mammal over a given time period; for example, weeks, months or years. In preferred practice, the pharmaceutical composition is administered to the mammal a number of times over a period of about thirty days.

A method for synthesizing the compounds is yet another aspect of the present invention. In accordance with one embodiment of this method, a compound comprising a trans-epoxide with a 7Z configuration that corresponds to the foregoing formula (wherein $R_1$ and $R_3$ taken together form an epoxide) is reacted with a substituted thiol. The resulting 5S-hydroxy-6S-thio derivative can be utilized as synthesized, or it can be further modified as by reaction with a suitable reagent, such as silver carbonate, to form a product wherein $R_2$ and $R_3$ of the foregoing formula taken together form the corresponding internal lactone.

In yet another embodiment, the above 5S-hydroxy-6S-thio derivative is oxidized with a suitable reagent, such as oxalyl chloride and DMSO, to form the corresponding 5-keto derivative which upon reaction with hydroxylamine hydrochloride, for example, provides the corresponding 5-oximino derivative.

The following Examples illustrate the preparation of a number of the compounds according to the present invention. By way of summary, the preferred compounds of this invention can be prepared in the following manner.

As shown in the following Reaction Sequence 1, a tetrahydropyranyl (THP)-protected heptanoate [Compound 1(a)] is reduced with diisobutylaluminum hydride (DIBAL-H) to produce the allylic alcohol [Compound 1(b)], which is in turn oxidized under Sharpless conditions (titanium isopropoxide, diethyl-L-tartrate and t-butyl hydroperoxide) to the trans-epoxy primary alcohol [Compound 1(c)].

Collins oxidation [Pyridine•$CrO_3$ complex] of the epoxy primary alcohol produces the corresponding epoxy aldehyde [Compound 1(d)], which is then treated with an appropriate Wittig reagent to form an ethylenic epoxide [Compound 1(e)]. Suitable Wittig reagents include dodecyl triphenyl phosphonium bromide and pentadecyltriphenyl phosphonium bromide. The phosponium salt is converted to an ylide by treatment with a strong base such as n-butyl lithium. The ylide reacts with the epoxy aldehyde to provide the trans-epoxide intermediate [Compound 1(e)] with a 7Z configuration.

Opening of the epoxide to a 5S-hydroxy-6S-thio derivative is accomplished using an appropriately substituted thiol in a triethylamine:methanol mixture as described in Examples 2-9 inclusive.

In those cases where $R_2$ is a hydroxyethyl group (as in Compounds 2, 4, 5, 7, 10-12, 14 and 16), the THP-protecting group can be removed by treatment with acid in a solvent such as methanol.

In addition to the THP-protecting group, other conventional hydrolyzable ether groups may be utilized to protect a hydroxy group. Among the preferred ether protecting groups are also included t-butyl and aryl methyl ethers. A preferred aryl methyl ether is a benzyl derivative.

## Reaction Sequence 1

Example No(s).

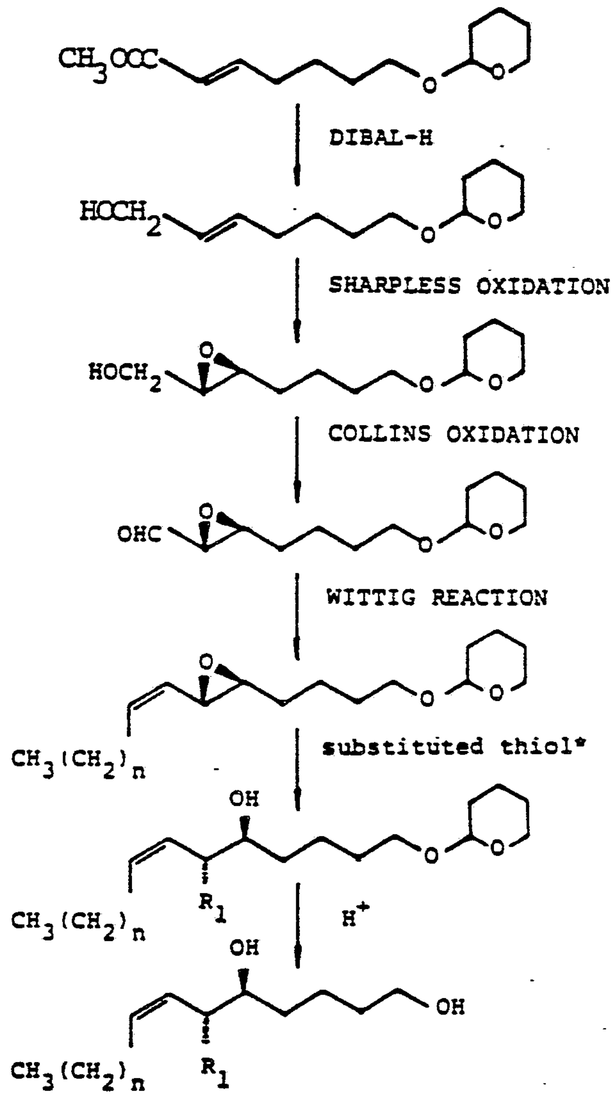


1(a)

1(b)

1(c)

1(d)

1(e)

3, 6, 8, 9 and 15

2, 4, 5, 7, 10 - 12, 16 and 20

* wherein $R_1$ represents the substituted thiol

Referring to the following Reaction Sequence 2, in those cases where $R_2$ is a hydroxyethyl group (Compounds 2, 4, 5, 7, 10-12, 14 and 16), the corresponding lactones can be formed upon treatment of the hydroxyethyl-functional compound with Fetizon's reagent (silver carbonate on Celite).

For example, the 1,5-diol (Compound 11) is treated with Fetizon's reagent to provide the lactone (compound 19). The addition of sodium hydroxide in tetrahydrofuran to the lactone provides the corresponding carboxylic acid salt (for example, Compound 20).

However, any conventional pharmaceutically acceptable salt can be used. Among the salts that can be prepared are alkali metal salts including lithium, sodium and potassium; alkaline earth metal salts including calcium and magnesium; and aluminum, zinc and iron. Also suitable are ammonium and substituted ammonium salts including those prepared from lower alkyl amines such as triethylamine; hydroxyalkylamines such as 2-hydroxyethylamine, tris(hydroxymethyl)aminomethane, bis-(2-hydroxyethyl)amine and tris-(2-hydroxyethyl)amine; cycloalkylamines such as bicyclohexylamine and bases of the pyridine type such as pyridine, collidine and quinoline.

Such salts can be formed from those compounds of the present invention which include a carboxylic acid group by treating the acid in a conventional manner with a base such as a metal hydroxide, ammonium hydroxide or an amine.

Since the compounds of the present invention can include more than one carboxylic acid group, partial salts or partial lower alkyl esters (compounds in which not all the carboxylic acid-functional groups are in salt or ester form) are contemplated, as are mixed salt/ester forms.

## Reaction Sequence 2

Example No(s).

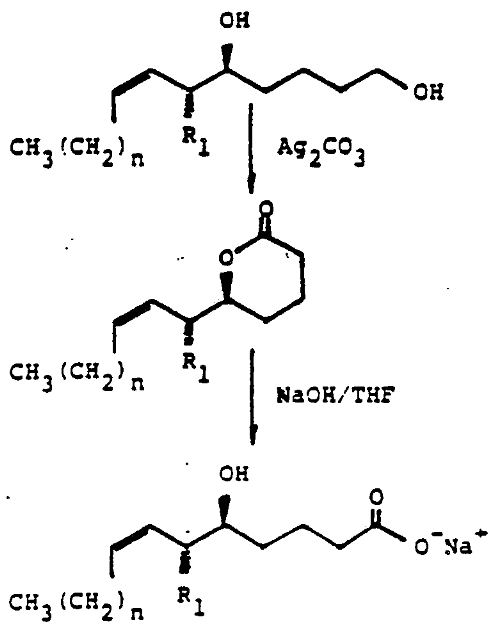


2, 4, 5, 7, 10 - 12

19

20

With reference to the following Reaction Sequence 3, a THP-protected diol (Compounds 3, 6, 8, 9 and 15) can be oxidized to form the corresponding THP-protected ketone.

For example the THP-protected diol (compound 6) is treated with oxalyl chloride/DMSO (dimethylsulfoxide) in methylene chloride at a reduced temperature to form the ketone (Compound 13). Reaction of the ketone under suitable conditions with hydroxylamine hydrochloride provides the corresponding oxime (Compound 14).

## Reaction Sequence 3

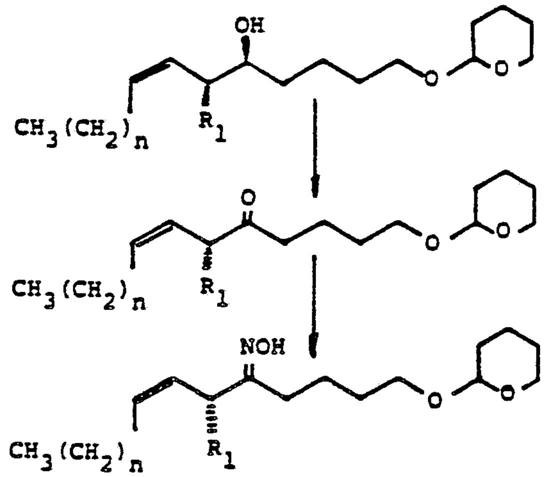

Example No(s).

3, 6, 8, 9 and 15

13

14

Having generally described the invention, a more complete understanding can be obtained by reference to certain examples, which are included for purposes illustrated only and are not intended to be limiting.

A series of compounds that were synthesized according to the method of the present invention are listed in table 1, hereinbefore. As used herein, the number assigned to each compound corresponds to the number of the following Example that describes the synthesis of that compound.

As will be appreciated, the compounds of this invention possess chiral centers at $C_5$ and $C_6$ and, accordingly, exist in the stereoisomeric forms 5R, 6R; 5S, 6S; 5R, 6S and 5S, 6R. Other chiral centers are also possible depending on the nature of the substituents $R_1$, $R_2$ and $R_3$. The compounds of the present invention, however, are not limited to any particular isomeric form.

### Best Mode of Carrying Out the Invention

In the following Examples, melting points (mp) were determined on a Thomas-Hoover apparatus, and the melting points reported herein are uncorrected. The infrared (IR) spectra were recorded on a Beckman Instruments IR-8 spectrophotometer and are expressed in reciprocal centimeters. Nuclear magnetic resonance (NMR) spectra for hydrogen atoms were obtained in $CDCl_3$ unless otherwise indicated with tetramethylsilane (TMS) as the internal standard on a Varian T-60A or an IBM WP-100 spectrometer. The values are expressed in parts per million downfield from TMS. Parenthesized, underlined hydrogens were assigned to the resonance positions immediately before the parentheses. Mass spectra were obtained on a Finnigan 1015D quadrupole mass spectrometer coupled to a Finnigan 9500 gas chromatograph or on a Finnigan MAT 8230 Double Focusing high resolution mass spectrometer.

### Example 1(a)

#### Preparation of Methyl-7-(2-tetrahydro-pyranyloxy-2(E)-heptenoate [Compound 1(a)]

To a solution of 17.98 grams of 5-hydroxyvaleraldehyde (176.03 mM) in 2.50 ml dry tetrahydrofuran (THF) under nitrogen were added 61.80 grams of methyl (triphenylphosphoranylidene)acetate (184.83 mM). The reaction mixture was heated to reflux for 4.5 hours and, after cooling to room temperature, was concentrated in vacuo to a white slurry. Diethyl ether (500 ml) and hexane (400 ml) were added, and the mixture was cooled to 0°C and was maintained at that temperature for 1 hour, at which time the solids were filtered and the filtrate was concentrated in vacuo to afford a further amount of precipitate. The total

precipitate provided 38.8 grams of crude methyl-7-hydroxy-2(E)-heptenoate (70% pure by NMR). A portion of the crude methyl heptenoate (2.0 grams) was chromatographed (Merck silica gel, 60, 100 grams) with a 2:1 mixture of hexane: diethyl ether as eluent to afford 1.38 grams of pure methyl-7-hydroxy-2(E)-heptenoate as a clear colorless liquid (96%).

To a solution of 7.0 grams of the above crude methyl heptenoate (30.84 mM) in 300 ml methylene chloride at 0°C under nitrogen were added 4.24 ml of dihydropyran (46.46 mM) and a catalytic amount (1 mg) of p-toluenesulfonic acid monohydrate. After stirring for 20 hours, 0.5 grams of sodium carbonate was added, the solid was filtered, and the reaction mixture was concentrated in vacuo to a clear brown liquid. A solid precipitated after 16 hours, 100 ml of hexane was added and the solution was filtered and concentrated in vacuo to afford 9.11 grams of crude methyl-7-(2-tetrahydropyranyloxy)-2(E)-heptenoate [Compound 1(a)]. A 2 gram portion of crude compound 1(a) was chromatographed (Merck silica gel 60, 200 grams) with a 1:19 triethylamine:hexane mixture as the eluent to afford 1.34 grams of pure compound 1(a) as a clear colorless liquid (82%).

NMR 1.2-2.0 (multiplet, 10 H, $-OCH_2CH_2CH_2CH_2CH=$ and $-OCH_2CH_2C\ H_2CH_2CH-$); 2.0-2.5 (multiplet, 2 H, $=CHC\ H_2CH_2-$); 3.1-4.1 (multiplet, 4 H, $CH_2CH\ _2OCHOCH_2CH_2-$); 3.68 (singlet, 3 H, $-COOC\ H_3$); 4.4-4.7 (multiplet, 1 H, -OCHO); 4.80 (distorted triplet, J=15 Hz, J=1 Hz, 1 H, $=CHCOOCH_3$); 6.97 (triplet, J=15 Hz, J=7 Hz, 1 H, $CH_2CH=$)

IR neat: 2950, 2870, 1730 and 1660;

MS: m/e 242 ($M^+$), 211, 210 and 101 and 85 (100%):

Anal. calculated for $C_{13}H_{22}O_4$: C, 64.43; H, 9.15

Found: C, 63.98; H, 9,05.

Example 1(b)

Preparation of 7-(2-tetrahydropyranyloxy)-2(E)-hepten-1-ol [Compound 1(b)]

To a solution of 5.00 grams (16.71 mM) of Compound 1(a) (82% pure by NMR) in 125 ml methylene chloride maintained at 0°C under nitrogen were added 41.8 ml diisobutylaluminum hydride (41.8 mM). After stirring of 0.5 hours, 5.0 ml methanol in 5.0 ml methylene chloride followed by 5.0 ml of a 1:1 water:methanol mixture were added to the reaction mixture. After gas evolution ceased, the mixture was filtered through a pad of Celite and was concentrated in vacuo to afford 3.65 grams of crude 7-(2-tetrahydropyranyloxy)-2(E)-hepten-1-ol [Compound 1(b)]. Chromatography (Merck silica gel, 60, 185 grams) with an 1:7:12 triethylamine:ethyl acetate:hexane mixture as eluent afforded 2.60 grams of pure compound 1(b) as a clear colorless liquid (73%).

NMR: 1.1-2.4 (multiplet, 13 H, $-OCH_2(CH_2)_3CH=$, $OCH_2(CH_2)_3CH-$and -OH); 3.1-4.0 (multiplet, 4 H, $-CH_2C\ H_2OCHOCH_2CH$); 3.8-4.3 (multiplet, 2 H, $=CHCH_2OH$); 4.4-4.7 (multiplet, 1 H, -OCHO-); 5.4-5.8 (multiplet, 2 H, -CH=CH-).

IR (neat): 3430, 2940, 2880 and 1445.

MS: m/e 214 ($M^+$), 196 ($M-H_2O$), 85 (100%).

Anal. calculated for $C_{12}H_{22}O_3$: C, 67.25; H, 10.35

Found: C, 67.08; H, 10.40.

Example 1(c)

Preparation of (2R,3S)-7-(2-tetrahydropyranyloxy)-2,3-trans-epoxyheptan-1-ol [Compound 1(c)]

Titanium (IV) isopropoxide (13.25 ml, 44.52 mM) and 9.18 grams (+)-diethyl L-tartrate (44.52 mM) were added via syringe to 400 ml methylene chloride maintained at -20°C under nitrogen. After 5 minutes, 9.54 grams (44.52 mM) of Compound 1(B) followed by 21.72 ml of 4.1 Molar (M) tert-butyl hydroperoxide (89.03 mM) were added via syringe. The reaction mixture was stirred at -20°C for 5 hours, stoppered and stored at -10°C for 16.5 hours. The mixture was cooled to -20°C and 100 ml of a 10% tartaric acid solution in water were added with vigorous stirring. The cooling bath was removed after 30 minutes, and stirring was continued for 1 hour. The layers were separated, and the organic layer was washed with water, dried over anhydrous sodium sulfate, filtered and concentrated in vacuo. The residue was diluted with 300 ml of diethyl ether and was cooled to 0°C. 1N sodium hydroxide (125 ml) was added, and the two-phase system was stirred for 30 minutes. The layers were separated, and the ether layer was washed with a saturated

sodium chloride solution, dried over anhydrous sodium sulfate, filtered and concentrated in vacuo to afford 8.17 grams of crude (2R, 3S)-7-(2-tetrahydropyranyloxy)-2-3-trans-epoxyheptan-1-ol [Compound 1(c)] as a clear yellow oil. Chromatography (Merck silica gel, 60, 700 grams) with a 1:6:13 triethylamine:ethyl acetate:hexane mixture as eluent afforded 4.29 grams of compound 1(c) as a clear light-yellow oil (42%).

NMR: 1.3-2.1 (multiplet, 12 H, $OCH_2(CH_2)_3CHO$ and $OCH_2(CH_2)_3CH$-; 2.2-2.5 (broad singlet, 1 H, -OH); 2.8-3.2 (multiplet, 2 H, epoxide Hs); 3.2-4.2 (multiplet, 6 H, $-CH_2OCHOCH_2$-and $-CH_2OH$); 4.5-4.7(multiplet, 1 H, -OCHO).

IR (neat): 3450 and 1260.

MS m/e 230 and 129.

Anal. Calculated for $C_{12}H_{22}O_4$: C, 62.58; H, 9.63 Found: C, 62.43; H, 9.58

Example 1(d)

Preparation of (2R,3S)-7-(2-tetrahydropyranyloxy)-2,3-trans-epoxyheptanal [Compound 1(d)]

Chromium trioxide (4.27 grams, 42.73 mM) was added in a single portion to a solution of 6.91 ml pyridine (85.44 mM) in methylene chloride. After stirring for 15 minutes, 1.64 grams (7.12 mM) of Compound 1(c) in 10 ml methylene chloride were added with a syringe. Stirring was continued for 30 minutes after which time the reaction mixture was filtered through a pad of Celite and was concentrated in vacuo. The dark brown residue was taken up in 100 ml diethyl ether and filtered through the same Celite pad. The filtrate was washed with 50 ml of a saturated copper sulfate solution, 50 ml of a saturated sodium chloride solution, dried over anhydrous sodium sulfate, filtered and concentrated in vacuo to afford 1.20 grams of crude (2R, 3S)-7-(tetrahydropyranyloxy)-2,3-trans-epoxyheptanal [Compound 1(d)] as a clear light-yellow oil. Chromatography (Merck silica gel, 60, 60 grams) with a 1:9:10 triethylamine:ethyl acetate:hexane mixture as euent afforded 0.95 grams of pure compound 1(d) as a clear colorless oil (58%).

NMR: 1.3-2.1 (multiplet, 12 H, $OCH_2(CH_2)_3CHO$-and $-OCH_2(CH_2)_3CH$-); 3.0-4.2 (multiplet, 6 H, $-CH_2OCHOCH_2$-and epoxide Hs); 4.5-4.7 (multiplet, 1 H, -OCHO); 9.03 (doublet, J=6 Hz, 1 H, -CHO).

IR (neat): 2730, 2660 and 1730.

MS: m/e 228 ($M^+$), 127 and 101.

Anal. calculated for $C_{12}H_{20}O_4$: C, 63.13; H, 8.83

Found: C, 63.04; H, 8.78.

Example 1(e)

Preparation of (5S,6S)-1-(2-Tetrahydropyranyloxy) -5,6-trans-epoxy-7(Z)-eicosene [Compound 1(e)]

N-Butyllithium (3.71 ml, 5.78 mM) was added with a syringe to a solution of 1.33 ml hexamethyldisilazane (6.31 mM) in 50 ml dry tetrahydrofuran (THF) at 0°C under nitrogen. After 15 minutes, 3.04 grams of powdered dodecyltriphenylphosphonium bromide (5.78 mM) were added in a single portion. 1.20 Grams (5.26 mM) of compound 1(d) in 5 ml dry THF was added 15 minutes later with a syringe. Stirring were continued for 1 hour after which time the reaction mixture was concentrated in vacuo to afford 5.62 grams of crude (5S,6S)-1-(2-tetrahydropyranyloxy)-5,6-trans-epoxy-7(Z)-eicosene [Compound 1(e)] as a brown viscous oil. Chromatography (Merck silica gel 60, 500 grams) with a 1:19 triethylamine:hexane mixture as eluent afforded 0.97 grams of pure compound 1(e) as a clear colorless oil (47%).

NMR: 0.7-1.0 (triplet, 3 H, $-CH_3$); 1.0-2.5 (multiplet, 34 H, $OCH_2(CH_2)_3CHO$, $-OCH_2(CH_2)_3CH$-and $=C-(CH_2)_{11}CH$); 2.6-3.0 (multiplet, 1 H, OCHCHC=); 3.0-4.1 (multiplet, 5 H, OCHCHC= and $-CH_2OCHOCH_2$-); 4.4-4.7 (multiplet, 1 H, -OCHO-); 4.7-5.4 (multiplet, 1 H, -OCHCH=CH); 5.68 (triplet, J=11 Hz and 7 Hz, 1 H, $-CH=CHCH_2$).

IR($CHCl_3$): 3020 and 1260.

MS: m/e 394 ($M^+$), 293 and 117 (100%).

Example 2

Preparation of Methyl N-acetyl-S-6[(5S,6R)-1,5-dihydroxy-7(Z)-eicosenyl]-(R)-cysteinate (Compound 2)

N-Acetylcysteine methyl ester (0.98 grams, 5.55 mM) and 1.55 ml triethylamine (11.10 mM) were added to a solution of 1.46 grams (3.70 mM) of Compound 1(e) in 35 ml methanol in the presence of nitrogen. After stirring overnight, the reaction mixture was concentrated in vacuo to afford methyl N-acetyl-S-6-[5S,6R]-5-hydroxy-1-(2-tetrahydropyranyloxy)-7(Z)-eicosenyl-(R)-cysteinate. The reaction mixture was diluted with 40 ml methanol, and a catalytic amount (1 mg) of p-toluenesulfonic acid was added. After stirring overnight, the reaction mixture was neutralized and dried over anhydrous sodium carbonate, filtered and concentrated in vacuo. The remaining residue was taken up in about 50 ml methylene chloride, filtered and concentrated in vacuo to afford 2.48 grams of crude methyl N-acetyl-S-6-[5S,6R]-1,5-dihydroxy-7(Z)-eicosenyl-(R)-cysteinate (Compound 2) as a clear yellow oil. Chromatography (Baker 40 µM silica gel, 75 grams) with a 1:9 methanol:methylene chloride mixture as eluent afforded 1.78 grams of compound 2 as a clear colorless oil (98.6%).

NMR: 0.88 (triplet, 3 H, $-CH_3$); 1.0-1.8 (multiplet, 26 H, $CH_3(CH_2)_{10}CH_2$ and $-CH(OH)(CH_2)_3CH_2OH$);1.8-2.3 (multiplet, 2 H, $CH_3(CH_2)_{10}CH_2CH=$); 2.07 (broad singlet, 5 H, $-NHCOCH_3$ and 2 -OH); 2.88 (broad doublet, J=6 Hz, 2 H, $-SCH_2CH-$); 3.3 - 3.9 (multiplet, 4 H, =CHCHCH(OH)-and $-CH_2OH$); 3.75 (singlet, 3 H, $-COOCH_3$); 4.5-5.0 (multiplet, 1 H, $CH_2CHNH$); 5.0-5.9 (multiplet, 2 H, -CH=CH-); 6.2-6.8 (multiplet, 1 H, -CH-NH-).

IR (neat): 3300, 1750 and 1660.

MS: m/e 385.

Anal. calculated for $C_{26}H_{49}NO_5S$: C, 64.02; H, 10.13; N, 2.87

Found: C, 63.87; H, 10.20; N, 3.09

Example 3

Preparation of S-6-[(5S,6R)-5-hydroxy-1-(2-tetrahydropyranyloxy)-7(Z)-eicosenyl] glutathione triethylammonium salt dihydrate (Compound 3)

To a 500 ml round bottom flask containing 0.50 grams (1.27 mM) of compound 1(e) in 15 ml methanol were added 1.17 grams (3.8 mM glutathione and 1.06 ml (7.60 mM) triethylamine. The reaction mixture was degassed and placed under nitrogen. After five days, the reaction mixture was concentrated in vacuo to afford 1.93 grams of crude S-6-[(5S,6R)-5-hydroxy-1-(2-tetrahydropyranyloxy)-7(Z)-eicosenyl] glutathione triethylammonium salt dihydrate (Compound 3). Chromatography (Baker 40 µM silica gel, 80 grams) with a 1:9:10 triethylamine:methanol:chloroform mixture as eluent afforded 0.94 grams of compound 3, mp = 140.5-143°C (88%).

NMR ($D_2O$): 0.85 (triplet, 3 H; $CH_3(CH_2)_{11}-$); 1.0-2.0 (multiplet, 41 H, $Ch_3(CH_2)_{10}CH_2-$, $-CH(OH)(CH_2)_3CH_2-$, $-OCH(CH_2)_3CH_2O$-and $(CH_3CH_2)_3N^+-$); 2.0-2.8 (multiplet, 6 H, $-CH_2CH=CH$-and $-COCH_2CH_2CH-$); 2.8-3.4 (multiplet, 2 H, $-SCH_2CH-$); 3.17 (quartet, J=7.5 Hz, 6 H, $(CH_3CH_2)_3N^+$); 3.4-4.3 (multiplet, 10 H, =CHCHCH-(OH)-, 2(-COCHNH-), $-NHCH_2COOH$ and $-CH_2OCHOCH_2-$); 4.4-4.6 (broad singlet, 1 H, -OCHO-); 4.67 (singlet, 7 H, -CH(OH)-, 2 (-COOH), 2(-NH-) and $-NH_2$); 5.0-6.2 (multiplet, 2 H, -CH=CH-).

IR ($CHCl_3$): 3300, 1650 and 1520.

MS: m/e 368.

Anal. calculated for $C_{35}H_{63}N_3O_9 \bullet C_6H_{15}N \bullet 2H_2O$: C, 58.68; H, 9.85; N, 6.68

Found: C, 58.73; H, 9.23; N, 6.50.

Example 4

Preparation of (5S,6R)-6-[S-(R)-cysteinyl] -7(Z)-eicosen-1,5-diol hemihydrate (Compound 4)

To a 50 ml round bottom flask containing 1.36 grams (3.45 mM) of Compound 1(e) in 25 ml methanol were added 0.46 grams cysteine (3.79 mM) and 1.08 ml triethylamine (7.75 mM). The reaction mixture was degassed and placed under nitrogen. After six days, the reaction mixture was concentrated in vacuo to afford crude S-6-[(5S,6R)-5-hydroxy-1-(2-tetrahydropyranyloxy)-7(Z)-eicos enyl] cysteine. Chromatography (Baker 40 µM silica gel, 75 grams) with a 1:2 methanol:chloroform mixture as eluent afforded 1.31 grams of the above cysteine intermediate as a white waxy solid (74%).

To a 250 ml round bottom flask containing 1.26 grams of the above cysteine intermediate (2.44 mM) in 25 methanol were added 3.60 ml of a 1N HCl solution (3.60 mM). After stirring overnight, the reaction mixture was neutralized with 1N NaOH solution (3.60 ml, 3.60 mM). After 1 hour, the reaction mixture was concentrated in vacuo. The remaining residue was partitioned between chloroform and water, the chloroform layer was dried over anhydrous sodium sulfate, filtered through a Celite pad and concentrated in vacuo to afford 1.04 grams of pure (5S,6R)-6-[S-(R)-cysteinyl]-7(Z)-eicosen-1,5-diol hemihydrate (Compound 4) as a white crystalline solid, mp = 140.5-143°C (97%).

Cysteine intermediate: NMR: 0.87 (triplet, 3 H, $CH_3(CH_2)_{11}$-); 1.0-1.8 (multiplet, 32 H, $CH_3(CH_2)_{10}$-$CH_2$-, -CH(OH)$(CH_2)_3CH_2$ and -OCH$(CH_2)_3CH_2O$-); 1.8-2.4 (multiplet, 2 H, $CH_3(CH_2)_{10}CH_2CH$=); 2.7-4.2 (multiplet, 8 H, =CHCHCH (OH)-, -$CH_2$OCHOCH$)_2$-and -COOH); 4.3-4.8 (multiplet, 2 H, -OCHO-and $H_2$NCHCOOH); 4.8-6.2 (multiplet, 2 H, -CH =CH-).

Compound 4: NMR ($CDCl_3$ and $CD_3OD$): 0.87 (triplet, 3 H, $CH_3CH_2$-); 1.0-1.8 (multiplet, 26 H, $CH_3(CH_2)_{10}CH_2$ and -CH(OH)$(CH_2)_3CH_2$); 1.8-2.4 (multiplet, 2 H, -$CH_2$-CH=); 2.7-3.3 (multiplet, 2 H, -S$CH_2$CH-); 3.4-4.0 (multiplet, 4 H, =CHCHCH(OH) and -$CH_2$OH); 4.07 (broad singlet, 5 H, 2(-OH), -COOH and -$NH_2$); 4.2-4.8 (multiplet, 1 H, $H_2$NCHCOOH); 5.0-6.0 (multiplet, 2 H, -CH=CH-).

IR (KBr): 3200, 1650 and 1580.

MS: m/e 369, 208, 103 and 85.

Anal. calculated for $C_{23}H_{45}NO_4S$•1/2$H_2O$: C, 62.68; H, 10.52; N, 3.18

Found: C, 62.27; H, 10.57; N, 3.59.

Example 5

Preparation of N-Acetyl-S-6-[(5S,6R)-1,5-dihydroxy-7(Z)-eicosenyl]-cysteamine (Compound 5)

To a 500 ml round bottom flask containing 1.40 grams (3.55 mM) of Compound 1(e) in 50 ml methanol were added 0.63 grams N-acetyl cysteamine (5.32 mM) and 1.48 ml triethylamine (10.64 mM). The solution was degassed and placed under nitrogen. After stirring overnight at room temperature, the solution was concentrated in vacuo to afford 2.06 grams of crude N-acetyl-S-6-[(5S,6R)-5-hydroxy-1-(2-tetrahydropyranyloxy)-7(Z)-eicosenyl]-cysteamine. Chromatography (Baker 40 µM silica gel, 80 grams) with a 1:14:5 triethylamine:ethyl acetate:hexane mixture as eluent afforded 1.69 grams of the above cysteamine intermediate as a clear colorless oil (93%).

To a 500 ml round bottom flask containing 1.64 grams (3.19 mM) of the above cysteamine intermediate in 40 ml methanol was added a catalytic amount (1 mg) of p-toluenesulfonic acid. After stirring overnight, the reaction mixture was neutralized with sodium carbonate, filtered and concentrated in vacuo. The remaining residue was taken up in methylene chloride, filtered through a Celite pad and concentrated in vacuo to afford 1.66 grams of crude N-acetyl-S-6-[(5S,6R)-1,5-dihydroxy-7(Z)-eicosenyl]-cysteamine (Compound 5). Chromatography (Baker 40 µM silica gel, 70 grams) with a 6:94 methanol:chloroform mixture as eluent afforded 1.34 grams of Compound 5 as a clear colorless oil, which was crystallized from diethyl ether/hexane to yield a white crystalline solid, mp = 46-48°C (98%).

Cysteamine intermediate: NMR: 0.87 (triplet, 3 H, $CH_3(CH_2)_{11}$-); 1.0-1.5 (multiplet, 20 H, $CH_3(CH_2)_{10}CH_2$-); 1.5-1.9 (multiplet, 12 H, -CH(OH)$(CH_2)_3CH_2O$-and -CH$(CH_2)_3CH_2O$); 1.9-2.3 (multiplet, 2 H, -$(CH_2)_{10}CH_2CH$=); 1.97 (singlet, 3 H, -NHCO$CH_3$); 2.4 (broad singlet, 1 H, -CH(OH)-); 2.60 (triplet, J=6 Hz, 2 H, -S$CH_2CH_2$-); 3.0-4.2 (multiplet, 8 H, =CHCHCH(OH)-, $CH_2$OCHOC $H_2$-and -$CH_2CH_2$NH-); 4.4-4.7 (multiplet, 1 H, -OCHO-); 5.0-6.3 (multiplet, 3 H, -CH =CH-and -NH-).

Compound 5: NMR: 0.90 (triplet, 3 H, $CH_3(CH_2)_{11}$-); 1.0-1.5 (multiplet, 20 H, $CH_3(CH_2)_{10}CH_2$-); 1.5-1.8 (multiplet, 6 H, -CH(OH)$(CH_2)_3$-$CH_2$); 1.8-2.3 (multiplet, 3 H, -$(CH_2)_{10}CH_2CH$=, and -$CH_2$(O H); 2.00 (singlet, 3 H, -NHCO$CH_3$); 2.4-2.8 (broad singlet, 1 H, -CH(OH)-); 2.63 (triplet, J=6 Hz, 2 H, -S-$CH_2$-$CH_2$-); 3.2-3.9 (multiplet, 6 H, =CH-CH-CH(OH)-, -$CH_2$OH, and -$CH_2$-$CH_2$-NH-); 5.0-6.3 (multiplet, 3 H, -CH =CH-and -NH-).

IR (neat): 3300 (-NH-, -OH), 1660 and 1555.

MS: m/e 327 ($M^+$-102), 221 and 208.

Anal. calculated for $C_{24}H_{47}NO_3S$: C, 67.08; H, 11.03; n, 3.26

Found: C, 66.99; H, 11.09; N, 3.24.

Example 6

Preparation of Methyl N-acetyl-S-6-[(5S,6R)-5-hydroxy-1-(2-tetrahydropyranyloxy)-7(Z)-eicosenyl]-(R)-cysteinate (Compound 6)

To a 50 ml round bottom flask containing 1.03 grams (2.61 mM) of Compound 1(e) in 25 ml methanol were added 0.46 grams of N-acetyl cysteine (2.61 mM) and 0.73 ml triethylamine (5.22 mM). The reaction mixture was degassed and placed under nitrogen. After stirring overnight, the reaction mixture was concentrated in vacuo. Chromatography of the reaction product (Baker 40 µM silica gel, 60 grams) with a 12:7:1 hexane:ethyl acetate:triethylamine mixture as eluent afforded 0.85 grams of pure methyl N-acetyl-S-6-[(5S,6R)-5-hydroxy-1-(2-tetrahydropyranyloxy)-7(Z)-eicosenyl-(R)-cysteinate (Compound 6) as a clear light yellow oil (57%).

NMR: 0.88 (triplet, 3 H, $C\underline{H}_3(CH_2)_{11}$-); 1.0-1.9 (multiplet, 32 H, $CH_3(C\underline{H}_2)_{10}$-, $-CH(OH)(C\underline{H}_2)_3CH_2$-and -CH-$(C\underline{H}_2)_3CH_2O$); 1.9-2.7 (multiplet, 3 H, $-CH_2C\underline{H}_2CH=$ and $-CH(O\underline{H})$-); 2.05 (singlet, 3 H, $-NHCOC\underline{H}_3$); 2.93 (doublet, J= 5 Hz, 2 H, $-SC\underline{H}_2CH$-); 3.1-4.1 (multiplet, 6 H, $=CHC\underline{H}C\underline{H}(OH)$-and $-C\underline{H}_2OCHOC\underline{H}_2$-); 3.75 (singlet, 3 H, $-COOC\underline{H}_3$); 4.4-4.7 (multiplet, 1 H, $-OC\underline{H}O$-); 4.7-5.0 (multiplet, 1 H, $-CH_2C\underline{H}NH$); 5.0-6.0 (multiplet, 2 H, $-C\underline{H}=C\underline{H}$-); 6.1-6.5 (multiplet, 1 H, $-N\underline{H}CO$-).

IR (neat): 3300, 1745 and 1670.

MS: m/e 488, 385, 342 and 326.

Anal. calculated for $C_{31}H_{57}NO_6S$: C, 65.11; H, 10.05; N, 2.45

Found: C, 65.18; H, 10.38; N, 2.44.

Example 7

Preparation of Methyl S-6-[(5S,6R)-1,5-dihydroxy-7(Z)-eicosenyl]-(R)-cysteinate (Compound 7)

To a 100 ml round bottom flask containing 1.93 grams (4.89 mM) of Compound 1(e) in 50 ml methanol were added 1.36 grams of cysteine ethyl ester hydrochoride (7.34 mM) and 3.07 ml triethylamine (22.0 mM). The reaction mixture was degassed and placed under nitrogen. After stirring for 3.5 days, the reaction mixture was concentrated in vacuo. The residue was chromatographed (Baker 40 µM silica gel, 140 grams) with a 97:3 chloroform:methanol mixture as eluent to afford 1.76 grams of ethyl S-6-[(5S,6R)-5-hydroxy-1-(2-tetrahydropyranyloxy)-7(Z)-eicosenyl-(R)-cysteinate as a clear yellow oil (66%).

To a solution of 1.76 grams (3.24 mM) of the above cysteinate in 30 ml methanol were added 4.80 ml of a 1N HCl solution (4.80 mM). After stirring for 18 hours, the reaction mixture was neutralized with 4.80 ml of a 1N sodium hydroxide solution (4.80 mM). After stirring for 1 hour, the reaction mixture was concentrated in vacuo. The residue was then partitioned between methylene chloride and water. The methylene chloride layer was dried over anhydrous sodium sulfate, filtered and concentrated in vacuo. The residue was chromatographed (Baker 40 µM silica gel, 70 grams) with a 1:19 methanol:chloroform mixture as eluent to afford 1.05 grams of ethyl S-6-[(5S,6R)-1,5-dihydroxy-7(Z)-eicosenyl]-(R)-cysteinate as a clear light-yellow oil (70%).

To a 500 ml round bottom flask containing 1.05 grams (2.28 mM) of the above ethyl cysteinate intermediate in 250 ml methanol were added 5.0 ml of 1N HCl solution (5.00 mM). After stirring for 2 days, the reaction mixture was neutralized with 5.0 ml of a 1N sodium hydroxide (5.00 mM) and stirred for 0.5 hours. The reaction mixture was concentrated in vacuo. The residue was partitioned between methylene chloride and water. The methylene chloride layer solution was dried over anhydrous sodium sulfate, filtered and concentrated in vacuo to afford 0.97 grams of methyl S-6-[(5S,6R)-1,5-dihydroxy-7(Z)-eicosenyl]-(R)-cysteinate (Compound 7) as a clear light-yellow oil (95%).

Compound 7: NMR: 0.87 (triplet, 3 H, $C\underline{H}_3(CH_2)_{10}$-); 1.1-1.8 (multiplet, 26 H, $CH_3(C\underline{H}_2)_{10}CH_2$-and -CH(OH)-$(C\underline{H}_2)_3CH_2OH$); 1.8-2.7 (multiplet, 6 H, $-(CH_2)_{10}C\underline{H}_2CH=$, $-N\underline{H}_2$ and 2 $-O\underline{H}$); 2.7-3.0 (multiplet, 2 H, $-SC\underline{H}_2CH$-); 3.3-4.0 (multiplet, 4 H, $=CHC\underline{H}C\underline{H}(OH)$-and $-C\underline{H}_2OH$); 3.73 (singlet, 3 H, $-COOC\underline{H}_3$); 5.2-6.0 (multiplet, 2 H, $-C\underline{H}=C\underline{H}$-).

IR (neat): 3360, 3300 and 1745.

MS: m/e 427, 394 and 342.

Anal. calculated for $C_{24}H_{47}NO_4S$: C, 64.67; H, 10.63; N, 3.14

Found: C, 64.94; H, 10.94; N, 3.06.

Example 8

Preparation of (5S,6R)-1-(2-tetrahydropyranyloxy)-5-hydroxy-6-phenylthio-7(Z)-eicosene 1/4 hydrate (Compound 8)

Thiophenol (0.30 ml, 2.89 mM), 1.21 ml triethylamine (8.67 mM) and 20 ml methanol were added to 0.57 grams (1.44 mM) of Compound 1(e) under nitrogen. After stirring for 24 hours, the reaction mixture was concentrated *in vacuo* to afford 0.92 grams of crude (5S, 6R)-1-(2-tetrahydropyranyloxy)-5-hydroxy-6-phenylthio-7(Z)-eicosene 1/4 hydrate (Compound 8). Chromatography (Merck silica gel 60, 75 grams) with a 1.9 triethylamine:hexane mixture as eluent afforded 0.67 grams of pure Compound 8 as a clear colorless oil (92%).

NMR: 0.7-1.0 (triplet, 3 H, $-CH_3$); 1.0-2.6 (multiplet, 35 H, $OCH_2(CH_2)_3CHO$, -OH, $-OCH_2(CH_2)_3CH$-and = $C(CH_2)_{11}CH_3$); 3.0-4.2 (multiplet, 6 H, $-CH_2OCHOCH_2$-and $-CH_2CH(OH)CH(SC_6H_5)$-); 4.4-4.7 (multiplet, 1 H, -OCHO-); 5.1-5.8 (multiplet, 2 H, -CH =CH-); 7.1-7.6 (multiplet, 5 H, aromatic Hs).

IR (neat): 3450 and 1120.

MS: m/e 504 ($M^+$), 403, 318 and 140 (100%).

Anal. calculated for $C_{31}H_{52}O_3S \bullet 1/4H_2O$: C, 73.10; H, 10.39 Found: C, 73.16; H, 10.22.

Example 9

Preparation of (5S,6R)-6-(4-chlorophenylthio)-5-hydroxy-1-(2-tetrahydropyranyloxy)-7(Z)-eicosene (Compound 9)

pChlorothiophenol (0.88 grams, 6.08 mM) and 1.70 ml triethylamine (12.16 mM) were added to a solution of 1.60 grams (4.05 mM) of Compound 1(e) in 40 ml methanol under nitrogen. After stirring overnight, the reaction mixture was concentrated *in vacuo* to afford 2.68 grams of crude (5S,6R)-6-(4-chlorophenylthio)-5-hydroxy-1-(2-tetrahydropyranyloxy)-7(Z)-eicosene (Compound 9) as a clear light-yellow oil. Chromatography (Baker 40 µM silica gel, 80 grams) with a 1:9 triethylamine:hexane mixture as eluent afforded 2.06 grams of pure Compound 9 as a clear colorless oil (94%).

NMR: 0.6-1.0 (multiplet, 3 H, $(CH_2)_{11}CH_3$); 1.0-2.2 (multiplet, 35 H, $=CH(CH_2)_{11}CH_3$), $-CH(OH)(CH_2)_3CH_2O$ and $OCH_2(CH_2)_3CHO$); 3.1-4.2 (multiplet, 6 H, =CHCHCH(OH)-and $-CH_2OCHOCH_2$-); 4.5-4.7 (multiplet, 1 H, -OCHO-); 5.1-5.8 (multiplet, 2 H, -CH =CH-); 7.0-7.5 (multiplet, 4 H, aromatic H).

IR (neat): 3440 and 1900.

MS: m/e 438, 436 (m-102), 352 and 85 (100%).

Anal. calculated for $C_{31}H_{51}ClO_3S$: C, 69.04; H, 9.53

Found: C, 69.15; H, 9.60.

Example 10

Preparation of (5S,6R)-6-[(R)-2-acetylamino-2-carbomethoxyethylsulfonyl]-7(Z)-eicosen-1,5-diol (Compound 10)

OXONE (3.82 grams, 6.21 mM $KHSO_5$) was added to a solution of 1.01 grams (2.07 mM) of Compound 2 in 15 ml methanol and 15 ml water. OXONE is a trademark of the DuPont Co., Wilmington, DE for a potassium peroxymonosulfate complex. After stirring overnight, the reaction mixture was concentrated *in vacuo*. The residue was diluted with methylene chloride, washed with water, dried over anhydrous sodium sulfate, filtered and concentrated *in vacuo* to afford 0.90 grams of (5S,6R)-6-[(R)-2-acetylamino-2-carbomethoxyethylsulfonyl]-7(Z)-eicosen-1,5-diol (Compound 10) as a clear light yellow oil (84%).

NMR: 0.88 (triplet, 3 H, $(CH_2)_{11}CH_3$); 1.0-1.8 (multiplet, 26 H, $=CHCH_2(CH_2)_{10}CH_3$ and $-CH(OH)(CH_2)_3CH_2OH$); 1.8-2.4 (multiplet, 2 H, $CH_3(CH_2)_{10}CH_2CH=$); 2.05 (broad singlet, 5 H, $-NHCOCH_3$ and 2 -OH); 3.5-4.0 (multiplet, 5 H, $-CH(OH)(CH_2)_3CH_2OH$ and $-SO_2CH_2CH$-); 3.77 (singlet, 3 H, $-COOCH_3$); 4.2-4.7 (multiplet, 1 H, =CHCHCH(OH)-); 4.7-5.2 (multiplet, 1 H, $-CH_2CH$ NH-); 5.3-6.4 (multiplet, 2 H, -CH=CH); 6.5-7.0 (multiplet, 1 H, -NH-).

IR (neat): 3660, 1750, 1665 and 1530.

MS: m/e 417 (M-102), 293, 192 and 145 (100%).

Anal. calculated for $C_{26}H_{49}NO_7S$: C, 60.08; H, 9.50; N, 2.70

Found: C, 60.20; H, 9.43; N, 2.78.

Example 11

Preparation of (5S,6R)-6-phenylthio-7(Z)-eicosen-1,5-diol (Compound 11)

A catalytic amount (1 mg) of p-toluenesulfonic acid was added to a solution of 1.06 grams (2.10 mM) of Compound 8 in 20 ml methanol. After stirring for 3 hours, the reaction mixture was neutralized and dried with sodium carbonate, filtered and concentrated *in vacuo*. The residue was taken up in 50 ml methylene chloride, filtered and concentrated *in vacuo* to afford 0.95 grams of crude (5S,6R)-6-phenylthio-7(Z)-eicosen-1,5-diol (Compound 11) as a clear colorless oil. Chromatography (Baker 40 µM silica gel, 30 grams) with a 1:19 methanol:methylene chloride mixture as eluent afforded 0.86 grams of Compound 11 as a clear colorless oil (97%).

NMR: 0.88 (triplet, 3 H, -C $\underline{H}_3$); 1.0-2.6 (multiplet, 30 H, $CH_3(C\underline{H}_2)_{11}CH=$ and $-CH(O\underline{H})(C\underline{H}_2)_3CH_2O\underline{H}$); 3.3-4.9 (multiplet, 3 H, -C$\underline{H}$ $(OH)(CH_2)_3(C\underline{H}_2OH)$; 4.0 (double doublet, J=4 Hz and 11 Hz, 1 H, =CHC$\underline{H}$CH(OH)-); 5.1-5.9 (multiplet, 2 H, -C $\underline{H}$=C$\underline{H}$-); 7.1-7.6 (multiplet, 5 H, aromatic Hs).

IR (neat): 3340 and 1020.

MS: m/e 318 (M-102) and 218 (100%).

Anal. calculated for $C_{26}H_{44}O_2S$: C, 74.23; H, 10.54

Found: C, 73.92; H, 10.31.

Example 12

Preparation of (5S,6R)-6-(4-chlorophenylthio)-7(Z)-eicosen-1,5-diol (Compound 12)

A catalytic amount (1 mg) of p-toluenesulfonic acid was added to a solution of 1.20 grams (2.23 mM) of compound 9 in 30 ml methanol. After stirring for 3 hours, the reaction mixture was neutralized and dried with sodium carbonate, filtered and concentrated *in vacuo*. The residue was taken up in 50 ml methylene chloride, filtered and concentrated *in vacuo* to afford 1.26 grams of crude (5S,6R)-6-(4-chlorophenylthio)-7-(Z)-eicosen-1,5-diol (compound 12) as a clear colorless oil. Chromatography (Baker 40 µM silica gel, 40 grams) with a 1:19 methanol:methylene chloride mixture as eluent afforded 1.02 grams of Compound 12 as a clear colorless oil (97%).

NMR: 0.6-1.1 (triplet, 3 H, -C$\underline{H}_3$); 1.1-2.6 (multiplet, 30 H, $CH_3(C\underline{H}_2)_{11}C=$ and $-CH(O\underline{H})(C\underline{H}_2)_3CH_2O$ $\underline{H}$); 3.3-3.9 (multiplet, 3 H, $-C\underline{H}(OH)(CH_2)_3C\underline{H}_2)OH$); 3.93 (double of doublets, J=4 and 9 Hz, 1 H, =CHC$\underline{H}$CH(OH)-); 4.8-6.0 (multiplet, 2 H, -C$\underline{H}$ =C$\underline{H}$-); 7.0-7.5 (multiplet, 4 H, aromatic Hs).

IR (neat): 3360 and 1100.

MS: m/e 354 (M+2-102, monoisotopic Cl), 352(M-102), 81 (100%).

Anal. calculated for $C_{26}H_{43}ClO_2S$: C, 68.61; H, 9.52

Found: C, 68.29; H, 9.64.

Example 13

Preparation of Methyl N-acetyl-S-6-[(5S,6R)-1-(2-tetrahydropyranyloxy)-5-oxo-7(Z)-eicosenyl]-(R)-cysteinate (Compound 13)

To a 50 ml round bottom flask containing 0.68 ml oxalyl chloride (7.77 mM) in 18 ml methylene chloride at -60°C (in a chloroform/dry ice slush bath) under nitrogen, were added 1.20 ml dimethyl sulfoxide (16.96 mM) in 4 ml methylene chloride. After stirring for 2 minutes, 2.02 grams (3.53 mM) of Compound 6 in 7 ml methylene chloride were added in a single portion. The -60°C cooling bath was replaced with a -12°C cooling bath (sodium chloride and ice water). After the reaction temperature reached -15°C, stirring was continued for 15 minutes, at which time 4.92 ml of triethylamine (35.3 mM) were added dropwise while maintaining the reaction temperature below -10°C. A heavy white precipitate formed. After stirring for 15 minutes, the reaction mixture was allowed to warm to room temperature.

The reaction mixture was washed with 40 ml of water. The water wash was extracted with 35 ml of methylene chloride and was combined with the reaction solution. The resulting organic solution was washed successively with 20 ml of a 1% HCl solution, 20 ml of a 5% sodium carbonate solution and 20 ml of a saturated sodium chloride solution. The solution was then dried over anhydrous sodium sulfate, filtered and

concentrated in vacuoto afford 2.26 grams of crude methyl N-acetyl-S-6-[[5S,6R)-1-(2-tetrahydropyranyloxy)-5-oxo-7(Z)-eicosenyl]-(R)-cysteinate (Compound 13) as a clear yellow oil. Chromatography (Baker 40 µM silica gel, 90 grams) with a 1:5:14 triethylamine:ethyl acetate:hexane mixture as eluent afforded 1.35 grams of pure compound 13 as a clear yellow oil (67%).

NMR: 0.92 (triplet, 3 H, $CH_3(CH_2)_{11}$-); 1.0-1.5 (multiplet, 20 H, $CH_3(CH_2)_{10}CH_2$-); 1.4-1.9 (multiplet, 10 H, -$COCH_2(CH_2)_2CH_2O$-and -$OCH(CH_2)_3CH_2O$); 2.05 (singlet, 3 H, -$COCH_3$); 1.9-2.4 (multiplet, 2 H, -$(CH_2)_{10}CH_2CH$=); 2.4-2.8 (multiplet, 2 H, -$COCH_2CH_2$-); 2.92 (doublet, J=5 Hz, 2 H, -$SCH_2CH$-); 3.0-4.1 (multiplet, 4 H, -$CH_2OCHOCH_2$-); 3.75 (singlet, 3 H, -$COOCH_3$); 4.30 (doublet, J=9 Hz, 1 H, =CHCHS-); 4.4-4.7 (broad singlet, 1 H, -OCHO-); 4.6-4.9 (multiplet, 1 H, -$CH_2CHNH$-); 5.0-6.0 (multiplet, 2 H, -CH=C H-); 6.0-6.6 (multiplet, 1 H, -NHCO-).

IR (neat): 3300, 1750, 1670, 1530 and 1030.

MS: m/e 467, 385, 144, 101 and 85 (100%).

Anal. calculated for $C_{31}H_{55}NO_6S$: C, 65.34; H, 9.73; N, 2.46

Found: C, 65.07; H, 9.96; N, 2.42.

## Example 14

### Preparation of Methyl N-acetyl-S-6-[(5S,6R)-1-hydroxy-5-oximino-7(Z)-eicosenyl]-(R)-cysteinate (Compound 14)

To a 250 ml round bottom flask containing 2.11 grams (3.70 mM) of compound 13 were added 0.27 grams of hydroxylamine hydrochloride (3.89 mM) and 50 ml ethanol. After stirring for 15 minutes, 0.60 ml pyridine (7.41 mM) were added, and the reaction mixture was cooled and concentrated in vacuo. The remaining residue was partitioned between methylene chloride and water. The organic layer was dried over anhydrous sodium sulfate, filtered and concentrated in vacuo to afford 2.01 grams of crude methyl N-acetyl-S-6-[(5S,6R)-1-hydroxy-5-oximino-7(Z)-eicosenyl-(R)-cysteinate (Compound 14). Chromatography (Merck silica gel 60, 200 grams) with a 3:1 ethyl acetate:hexane mixture as eluent afforded 0.57 grams of pure Compound 14 as a clear colorless oil (31%).

NMR: 0.88 (triplet, 3 H, $CH_3(CH_2)_{11}$-); 1.0-1.5 (multiplet, 20 H, $CH_3(CH_2)_{10}CH_2$-); 1.4-1.9 (multiplet, 4 H, -N=$CCH_2(CH_2)_2CH_2OH$); 1.9-2.7 (multiplet, 6 H, -$(CH_2)_{10}CH_2CH$=, 2 -OH and -N=$CCH_2CH_2$-); 2.05 (singlet, 3 H, -$NHCOCH_3$); 2.88 (broad doublet, J=5 Hz, 2 H, -$SCH_2CH$-); 3.4-3.9 (multiplet, 2 H, -$CH_2OH$); 3.75 (singlet, 3 H, -$COOCH_3$); 4.42 (doublet of doublets, J=9 Hz and 2 Hz, 1 H, -CH=CHCH-); 4.5-5.1 (multiplet, 1 H, -$CH_2$-CH-NH-); 5.1-6.0 (multiplet, 2 H, -CH=CH-); 6.4-6.8 (multiplet, 1 H, -NH -).

IR (neat): 3280, 1745, 1660 and 1540.

MS: m/e 500 (M+), 325, 176, 169, 144 and 85 (100%).

Anal. calculated for $C_{26}H_{48}N_2O_5S$: C, 62.36; H, 9.66; N, 5.60

Found: C, 62.60; H, 10.01; N, 5.30.

## Example 15

### Preparation of Methyl N-acetyl-S-6-[(5S,6R)-5-hydroxy-1-(2-tetrahydropyranyloxy)-7(Z)-docosenyl]-(R)-cysteinate (Compound 15)

To a 100 ml round bottom flask containing 1.29 ml hexamethyldisilazane (5.78 mM) in 50 ml dry tetrahydrofuran at 5°C were added 3.56 ml n-butyllithium (5.52 mM). The cooling bath was removed and after 15 minutes 3.20 grams of pentadecyltriphenylphosphonium bromide (5.78 mM) were added in a single portion. The reaction mixture turned a deep red color. After 20 minutes, 1.20 grams (5.26 mM) of Compound 1(d) were added dropwise over a 5 minute period, and the reaction mixture turned yellow. Stirring was continued for 30 minutes, at which time the reaction solution was concentrated in vacuo. The residue was dissolved in a 1:1 diethyl ether:hexane mixture, filtered and concentrated in vacuo. Chromatography (Baker 40 µM silica gel, 100 grams) with a 1:19 triethylamine:hexane mixture as eluent afforded 1.72 grams of (5S,6R)-1-(2-tetrahydropyranyloxy)-5,6-trans-epoxy-7(Z)-docosene as a clear colorless oil (77%).

To a 500 ml round bottom flask containing 1.69 grams of the above docosene intermediate (4.00 mM) in 50 ml methanol were added 0.81 grams N-acetyl cysteine methyl ester (4.60 mM) and 1.39 ml triethylamine (10.00 mM). The reaction mixture was degassed and placed under nitrogen. After stirring for 5 days, the reaction mixture was concentrated in vacuo. The residue was chromatographed (Baker 40 µM silica gel, 100 grams) with a 10:9:1 hexane:ethyl acetate:triethylamine mixture as eluent to afford 2.04 grams of methyl N-acetyl-S-6-[(5S, 6R)-5-hydroxy-1-(2-tetrahydropyranyloxy)-7(Z)-docosenyl]-(R)-cysteinate (Compound 15) as a clear colorless oil (85%).

Docosene intermediate: NMR 0.7-1.0 (triplet, 3 H, -C$\underline{H}_3$); 1.0-2.5 (multiplet, 38 H, $CH_3(C\underline{H}_2)_{13}CH$=, -CH-$(C\underline{H}_2)_3CH_2O$-and -OCH$(C\underline{H}_2)_3CH_2O$); 2.6-3.0 (multiplet, 1 H, =CHCHOC$\underline{H}$-); 3.0-4.1 (multiplet, 5 H, =CHC$\underline{H}$OCH-and -C$\underline{H}_2$OCHO-C$\underline{H}_2$); 4.4-4.7 (multiplet, 1 H, -OC$\underline{H}$O-); 4.7-5.4 (multiplet, 1 H, -CH=C$\underline{H}$CH-); 5.68 (triplet, J=11 Hz and 7 Hz, 1 H, -$CH_3$C $\underline{H}$=CH-).

Compound 15: NMR: 0.85 (triplet, 3 H, C $\underline{H}_3(CH_2)_{13}$-); 1.0-1.5 (multiplet, 24 H, $CH_3$(C $\underline{H}_2)_{12}CH_2$-); 1.4-1.8 (multiplet, 12 H, -CH(OH)(C $\underline{H}_2)_3CH_2$-and -OCHOC$H_2$(C$\underline{H}_2)_3$); 1.8-2.5 (multiplet, 3 H, -C$\underline{H}_2$CH=CH-and -CH(O $\underline{H}$)-); 2.03 (singlet, 3 H, -NHCOC$\underline{H}_3$); 2.92 (doublet, J=5 Hz, 2 H, -SC$\underline{H}_2$CH-); 3.1-4.1 (multiplet, 6 H, =CHC$\underline{H}$C$\underline{H}$(OH)-and -C$\underline{H}_2$OCHOC $\underline{H}_2$-); 3.72 (singlet, 3 H, -COOC$\underline{H}_3$); 4.4-4.7 (multiplet, 1 H, -OC$\underline{H}$O-); 4.7-5.1 (multiplet, 1 H, -$CH_2$C$\underline{H}$NH-, 5.2-6.9 (multiplet, 2 H, -C$\underline{H}$=C $\underline{H}$-); 6.30 (broad doublet, J=9 Hz, 1 H, -N$\underline{H}$-).

IR (neat): 3300, 1750 and 1665.

MS: m/e 413, 370, 354, 321 and 236.

Anal. calculated for $C_{33}H_{61}NO_6S$: C, 66.07; H, 10.25; N, 2.34

Found: C, 65.88; H, 10.16; N, 2.38.

## Example 16

### Preparation of Methyl N-acetyl-S-6-[(5S,6R)-1,5-dihydroxy-7(Z)-docosenyl]-(R)-cysteinate (Compound 16)

To a 100 ml round bottom flask containing 1.38 grams of the cysteinate of Example 15 (2.30 mM) in 25 ml methanol was added 1 mg of p-toluenesulfonic acid. After stirring for six days, the reaction mixture was concentrated in vacuo. Chromatography (Baker 40 µM silica gel, 55 grams) of the residue with a 1:19 methanol:chloroform mixture as eluent afforded 1.15 grams of methyl N-acetyl-S-6-[(5S,6R)-1,5-dihydroxy-7(Z)-docosenyl]-(R)-cysteinate (Compound 16) as a clear colorless oil (97%).

NMR: 0.87 (triplet, 3 H, C$\underline{H}_3(CH_2)_{13}$-); 1.0-1.8 (multiplet, 30 H, $CH_3(C\underline{H}_2)_{12}CH_2$-and -CH(OH)(C$\underline{H}_2)_3CH_2$OH); 1.8-2.3 (multiplet, 2 H, -C$\underline{H}_2$CH=CH-); 2.05 (singlet, 3 H, -NHCOC$\underline{H}_3$); 2.33 (singlet, 2 H, 2 -O $\underline{H}$); 2.90 (broad doublet, J=5 Hz, 2 H, -SC$\underline{H}_2$CH); 3.4-3.9 (multiplet, 4 H, =CHC$\underline{H}$C$\underline{H}$(OH)-and -C$\underline{H}_2$OH); 3.75 (singlet, 3 H, -COOC$\underline{H}_3$); 4.6-5.1 (multiplet, 1 H, -$CH_2$-C$\underline{H}$-NH-); 5.1-6.0 (multiplet, 2 H,-C$\underline{H}$=C$\underline{H}$-); 6.2-6.7 (multiplet, 1 H, -N$\underline{H}$-).

IR (neat): 3300, 1750, 1660 and 1540.

MS: m/e 413, 370, 354, 321 and 236.

Anal. calculated for $C_{28}H_{53}NO_5S$: C, 65.20; H, 10.36; N, 2.72

Found: C, 64.81; H, 10.25; N, 3.00.

## Example 17

### Preparation of (6S,7S)-2,6-dimethyl-6,7-trans-epoxyheneicosa-2,8-(Z)-diene monohydrate (Compound 17)

A solution of 1.68 ml diisopropylamine (12mM) in 125 ml tetrahydrofuran was treated with 6.41 ml n-butyllithium (1.56 M, 10.0 mM) at room temperature. After 10 minutes, 5.43 grams of tridecyltriphenylphosphonium bromide (10.3 mM) were added in a single portion. The resulting red solution was cooled to -70° and 1.68 grams (10.0 mM) of (2R,2S)-3,7-dimethyl-6-ene-2,3-trans-epoxyoctanal dissolved in 5 ml THF were added. The reaction mixture was stirred for 1 hour at -70°C, and then allowed to warm to ambient temperature and stirred for 40 minutes. The mixture was concentrated in vacuo, and the residue was washed with several 50 ml portions of hexane. The combined washes concentrated to a light yellow oil which contained some solid material (2.13 grams). Chromatography of the crude product on Merck silica gel 60 (200 grams) and elution with an 8:2:1 mixture of hexane:diethyl ether:triethylamine afforded 1.83 grams of (6S,7S)-2,6-dimethyl-6,7-trans epoxyheneicosa-2,8-(Z)-diene monohydrate (Compound 17) as a very light liquid (55%).

NMR: 0.90 (triplet, 3 H, $CH_3CH_2$); 1.30 (broad singlet, 25 H, 6-$CH_3$, 5-$CH_2$ and $CH_3(CH_2)_{10}$); 1.60 and 1.70 (broad singlet, 6 H, $C=C(CH_3)_2$); 1.8-2.2 (multiplet, 4 H, 10-$CH_2$ and 4-$CH_2$), 3.40 (doublet, J=7 Hz, 1 H, J=7 Hz, $C_7$-H); 5.1 (broad triplet, J=6 Hz, 1 H, $C_3$-H ); 5.1-6.0 (multiplet, 2 H, $C_8$-H and $C_9$-H).

IR ($CHCl_3$): 2530, 2860 and 1460.

MS: m/e 334 ($M^+$), 291, 265, 109 (100%);

$[\alpha]_D^{22}$ (ethyl alcohol) = +24.5°.

Anal. calculated for $C_{12}H_{42}O{\bullet}H_2O$: C, 78.34; H, 12.58

Found: C, 78.21; H, 12.00.

## Example 18

### Preparation of (6S,7R)-2,6-dimethyl-6-hydroxy-7-phenylthiaheneicosa-2,8-(Z)-dienehemihydrate (Compound 18)

A solution of 1.00 grams (3.0 mM) of Compound 17 in 30 ml methanol containing 2.50 ml triethylamine (18 mM) was treated with 0.95 ml thiophenol (9.0 mM) at room temperature. The mixture was degassed and stirred overnight and then concentrated to a viscous oil (1.90 grams). The crude product was chromatographed on 175 grams of Merck silica gel 60, and eluted with a 9:1 hexane:diethyl ether mixture to afford 1.32 grams of (6S,7R)-2,6-dimethyl-6-hydroxy-7-phenylthiaheneicosa-2,8-(Z)-diene hemihydrate (Compound 18) as a light-yellow viscous oil (99%),

NMR: 0.85 (triplet, 3 H, -$CH_3$); 0.9-1.4 (broad singlet, 24 H, $(CH_2)_{10}$, 6 -$CH_3$ and -OH); 1.65 and 1.70 (broad singlet, 6 H, $C=C(CH_3)_2$); 1.8-2.4 (multiplet, 4 H, 10-$CH_2$ and 4-$CH_2$); 3.95 (complex doublet, J=10 Hz, 1 H, $C_7$-H); 5.15 (broad triplet, J=5 Hz, 1 H, $C_3$-H); 5.50 (doublet of doublets, J=10 Hz and 7.5 Hz, $C_8$-H and $C_9$-H); 7.0-7.6 (multiplet, 5 H, aromatic H).

IR ($CHCl_3$): 3450-3650, 2920 and 2850.

MS: m/e No $M^+$, 354, 334, 318, 291, 218 (100%), 185 and 155; $[\alpha]_D^{26}$ ($CHCl_3$) = +52.8°.

Anal. calculated for $C_{29}H_{48}SO{\bullet}1/2\ H_2O$: C, 76.76; H, 10.89

Found: C, 76.76; H, 10.19.

## Example 19

### Preparation of (5S,6R)-5-hydroxy-6-phenylthio-7(Z)-eicosenoic acid lactone (Compound 19)

Benzene (500 ml) was added to 92.8 grams of Fetizon's reagent (silver carbonate on Celite, 148.5 mM silver carbonate) in a one liter round bottom flask protected from light. The reaction mixture was heated to remove residual water from the reaction by azeotropic distillation with benzene. Distillate was collected up to 80°C. Compound 11 (4.16 grams, 9.90 mM) in 25 ml benzene was added to the reaction mixture. After stirring at reflux for 24 hours, the reaction mixture was allowed to cool to room temperature, filtered through a Celite pad and concentrated in vacuo to afford 4.19 grams of crude (5S,6R)-5-hydroxy-6-phenylthio-7(Z)-eicosenoic acid lactone (Compound 19). Chromatography (Merck silica gel 60, 70-230 mesh, 200 grams) with an ethyl acetate:hexane mixture (1:9 to 1:3) as eluent afforded 1.43 grams of Compound 19 as a clear light yellow oil (35%).

NMR: 0.87 (triplet, 3 H, $CH_3(CH_2)_{11}$-); 0.9-1.6 (multiplet, 20 H, $CH_3(CH_2)_{10}CH_2$-); 1.6-2.3 (multiplet, 6 H, $CH_3(CH_2)_{10}CH_2CH=$ and $-CH(CH_2)_2CH_2-COO$); 2.3-2.7 (multiplet, 2 H, $-CH(CH_2)_2CH_2COO$); 5.2-5.7 (multiplet, 2 H, -CH=CH-); 7.1-7.6 (multiplet, 5 H, aromatic H).

IR (neat): 1750 and 1240.

MS: m/e 416 ($M^+$), 307 and 317 (100%).

Anal. calculated for $C_{26}H_{40}O_2S$: C, 74.95; H, 9.68

found: C, 75.27; H, 9.69.

## Example 20

Preparation of Sodium (5S,6R)-5-hydroxy-6-phenylthio-7(Z)-eicosenoate hemihydrate (Compound 20)

A 1N sodium hydroxide solution (2.55 ml, 2.55 mM), 5.0 ml distilled water and 1.0 ml tetrahydrofuran were added to 1.06 grams (2.55 mM) of Compound 19. After stirring for 1 hour at room temperature, the reaction mixture became homogeneous, and was diluted with 15 ml water. The THF was removed by distillation. Lyophilization of the remaining aqueous solution afforded 1.14 grams of pure sodium (5S,6R)-5-hydroxy-6-phenylthio-7(Z)-eicosenoate hemihydrate (Compound 20) as a white solid (98%), mp = 216-217.5°C (discoloration started at 190°C).

NMR: 0.88 (triplet, 3 H, $C\underline{H}_3(CH_2)_{11}$-); 0.9-1.5 (multiplet, 24 H, $CH_3(C\underline{H}_2)_{10}CH_2$-and $-CH(OH)(C\underline{H}_2)_2CH_2$-); 1.5-2.0 (multiplet, 3 H, $CH_3(CH_2)_{10}C\underline{H}_2CH=$ and $-O\underline{H}$); 2.0-2.5 (multiplet, 2 H, $-CH_2C\underline{H}_2COO$); 3.5-4.2 (multiplet, 2 H, $=CHC\underline{H}C\underline{H}(OH)$-); 5.0-5.7 (multiplet, 2 H, $-C\underline{H}=C\underline{H}$-); 7.0-7.6 (multiplet, 5 H, aromatic H).

IR (KBr): 3400 (-OH) and 1560 (C=O).

Anal. calculated for $C_{26}H_{41}O_3SNa$ 1/2 $H_2O$: C, 67.06; H, 9.09

Found: C, 66.77; H, 8.85.

## Results

The following procedure is a modification of the Phospholipase $A_2$ assay that was first described by Costentino et al., Prostaglandins , 22, 309 (1981) and is the method used herein to determine rat stomach and porcine pancreas phospholipas $A_2$ activity.

A suspension of phospholipase $A_2$ (Sigma Chemical Co., St. Louis, MO) is diluted with 25 millimolar (mM) Tris HCl buffer containing 1 mM calcium chloride to a final concentration of 10 micrograms of protein per milliliter (ml). Aliquots of 0.1 ml (7.1 units) of the enzyme suspension are added to each assay tube. Test compounds are added tin 0.01 ml aliquots in dimethyl sulfoxide or methanol and the volume is brought to 0.15 ml with the buffer. A preincubation is carried out at 37°C for 5 minutes. To start the reaction, 0.01 microcuries (0.179 nanomoles) of 2-arachidonyl (1-$^{14}$C) 1-palmitoyl phosphatidyl choline (New England Nuclear, Boston, MA) are added to each tube, including three blank tubes containing no enzyme, and the incubation is carried out for 10 minutes at 37°C. The reaction is stopped with the addition of 0.07 ml of 1N HCl to each tube. Blanks, controls and each concentration of test compound are assayed in triplicate.

The reaction mixtures are extracted with ethyl acetate, and the organic and aqueous phases are separated by low speed centrifugation. The organic phase is dried under nitrogen and is then resuspended in 0.01 ml ethyl acetate. Arachidonic acid (50 micrograms in 0.01 ml methanol) is added to each tube as a carrier and standard. The entire mixture is spotted on silica gel G thin layer chromatography plates that are developed in a solvent system of ethyl acetate:acetic acid (99:1 by volume). Areas containing the unreacted phospholipid substrate (the origin) and unesterified arachidonic acid (near the solvent front) are scraped into separate scintillation vials. Ten ml of Aquasol-2 (American Cyanamid Co., Wayne, NJ) are added to each vial, and the radioactivity is counted by liquid scintillation spectrometry.

The data are calculated as the ratio in percent of unesterified arachidonic acid to total radioactivity recovered. The potency of a compound is preferably determined by its $IC_{50}$ value (the concentration required to inhibit the reaction by 50 percent). Usually, however, in the absence of an extensive inhibition study, the potency of a compound is reported as the percent inhibition of hydrolytic activity at a particular concentration of the compound.

Table 2, hereinafter, shows the percent inhibition of the hydrolytic activity of phospholipase $A_2$ obtained from rat stomach and porcine pancreas tissue samples by Compounds 2, 3, 4 and 14.

Compound 2 --Methyl N-acetyl-S-6-[(5S, 6R)-1,5-dihydroxy-7(Z)-eicosenyl]-(R)-cysteinate.

Compound 3 --S-6-[(5S, 6R)-5-hydroxy-1-(2-tetrahydropyranyloxy)-7(Z)-eicosenyl]-glutathione triethylammonium salt dihydrate.

Compound 4 --(5S, 6R)-6-[S-(R)-cysteinyl]-7(Z)-eicosen-1,5-diol hemihydrate.

Compound 14 --Methyl N-acetyl-S-6[(5S, 6R)-1-hydroxy-5-oximino-7(Z)-eicosenyl)]-(R)-cysteinate.

TABLE 2

Percent Inhibition* of the Hydrolytic Activity of

| Compound | Rat Stomach Phospholipase $A_2$ | Porcine Pancreas Phospholipase $A_2$ |
|---|---|---|
| 2 | 50 percent ($IC_{50}$) at $6.5 \times 10^{-6}$ Molar (M) | 62, 86 and 68 percent** at $10^{-5}$ Molar (M) |
| 3 | 55 and 57 percent at $10^{-4}$M | |
| 4 | 40 and 48 percent at $10^{-4}$M | |
| 14 | 58 percent at $10^{-4}$M | 72 and 73 percent at $10^{-4}$M |

*Percent inhibition of the identified phospholipase $A_2$ by a particular compound was determined according to the foregoing procedure.

**The results of three independent trials are reported.

Compounds that are generally used as standards to compare the activities of potential $PA_2$ inhibitors are listed below together with the corresponding $IC_{50}$ value from the literature for each compound and the particular source of the $PA_2$.

| Standard Compound | Structure | $IC_{50}$ |
|---|---|---|
| Chloroquin | 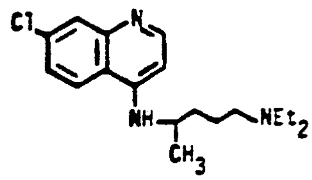 | $1.25x10^{-4}M$ (Rat stomach $PA_2$) |
| Mepacrin | 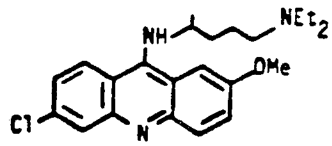 | $2.4x10^{-4}M$ (Hog pancreas $PA_2$) |
| U-10029A | 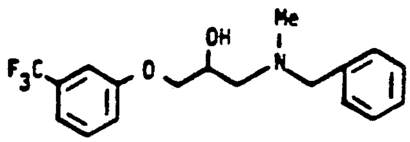 | $1.6x10^{-4}M$ (Hog pancreas $PA_2$) $4.1x10^{-4}M$ (Cobra venom $PA_2$) $4.9x10^{-4}M$ (Bee venom $PA_2$) |

Comparison of the activity of Compound 2, for example, to the reported activities for each of the above "standard" compounds demonstrates that Compound 2 is a more potent inhibitor of $PA_2$ activity than are the compounds that are usually used as standards.

Compound 2 was also studied in a mouse ear edema assay to provide a measure of topical anti-inflammatory activity.

The topical antiinflammatory activity of a compound is determined by its ability to inhibit an experimentally-induced inflammation in the mouse ear. Croton oil, or 12-0-tetradecanoylphorbol acetate in acetone is dispensed onto the dorsum of one ear of the mouse. Five to six hours later the mice are sacrificed. A section of each ear is removed with a 7 mm cork borer and weighed on a electronic balance. The difference in weight between treated and untreated ears is determined. Compounds to be evaluated for antiinflammatory activity are incorporated in the croton oil irritant mixture, or applied in an appropriate vehicle.

The percent inhibition at the following doses was observed.

| Compound 2 (micrograms) | Inhibition (percent) | |
|---|---|---|
| | Mouse No. 1 | Mouse No. 2 |
| 50 | 33 | 68 |
| 100 | 53 | 72 |
| 200 | 62 | 82 |
| 400 | 75 | 80 |

The present invention has been described with reference to several preferred embodiments. It will be understood, however, that numerous modifications and variations of the disclosed subject matter can be made without departing from the scope of the invention described herein.

## Claims

1. A compound having a structure that corresponds to the formula:

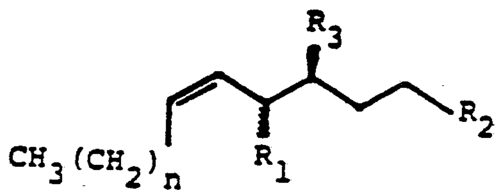

wherein $R_1$ is selected from the group consisting of unsubstituted or substituted thiophenyl, unsubstituted or substituted phenylsulfonyl, cysteinyl, glutathionyl, cysteinylglycinyl, unsubstituted or substituted lower thioalkylamino, unsubstituted or substituted sulfonylalkylamino, unsubstituted or substituted lower thioalkylacetamido and unsubstituted or substituted sulfonylalkyl-acetamido radicals;

$R_2$ is selected from the group consisting of lower alkyl hydroxy, lower alkyl tetrahydropyranyloxy, lower alkyl carboxy and lower alkenyl radicals;

$R_3$ is selected from the group consisting of hydroxy, carbonyl, oximino and lower alkyl radicals; and

n is an integer from about 10 to about 14; and the pharmaceutically acceptable salts, esters, epoxides and internal lactones thereof wherein $R_1$ and $R_3$ taken together form an epoxy group or wherein $R_2$ and $R_3$ taken together form a lactone.

2. The compound according to claim 1 wherein $R_1$ is substituted lower thioalkyl-acetamido, $R_2$ is lower alkyl hydroxy, $R_3$ is hydroxy and n is 11.

3. The compound according to claim 1 wherein $R_1$ is glutathionyl, $R_2$ is lower alkyl tetrahydropyranyloxy, $R_3$ is hydroxy and n is 11.

4. The compound according to claim 1 wherein $R_1$ is cysteinyl, $R_2$ is lower alkyl hydroxy, $R_3$ is hydroxy and n is 11.

5. The compound according to claim 1 wherein $R_1$ is substituted lower thioalkyl-acetamido, $R_2$ is lower alkyl hydroxy, $R_3$ is oximino and n is 11.

6. The compound according to claim 1 wherein $R_1$ is para-chlorothiophenyl.

7. The compound according to claim 1 wherein $R_1$ is $CH_2CH(COOCH_3)NHCOCH_3$.

8. The compound according to claim 1, claim 6 or claim 7 wherein $R_2$ is 2-hydroxyethyl.

9. The compound according to claim 1, claim 6 or claim 7, wherein $R_2$ is (2-tetrahydropyranyloxy) ethyl.

10. The compound according to claim 1 or any one of claims 6 to 9 wherein $R_3$ is hydroxy.

11. The compound according to claim 1 or any one of claims 6 to 10, wherein n is 11 or 13.

12. The compound according to claim 1 which is:

methyl N-acetyl-S-6-[5S,6R)-1,5-dihydroxy-7(Z)-eicosenyl]-(R)-cysteinate;

S-6-[(5S,6R)-5-hydroxy-1-(2-tetrahydropyranyloxy)-7(Z)-eicosenyl] glutathione triethylammonium salt dihydrate;

(5R,6R)-6-[S-(R)-cysteinyl]-7(Z)-eicosen-1,5-diol hemihydrate; or

methyl N-acetyl-S-6[(5S,6R)-1-hydroxy-5-oximino-7(Z)-eicosenyl)]-(R)-cysteinate.

13. A pharmaceutical composition comprising an anti-inflammatory amount of a compound according to any one of claims 1 to 12 as the active ingredient dispersed in a pharmaceutically acceptable carrier.

14. The pharmaceutical composition according to claim 13 wherein said carrier is an aerosol.

15. The pharmaceutical composition according to claim 13 wherein said carrier is selected from a lotion, cream or ointment.

16. The pharmaceutical composition according to any one of claims 13 to 15 wherein said compound is capable of inhibiting the hydrolytic activity of phospholipase $A_2$ in the amount present in the composition when said composition is introduced into a mammal.

17. A compound according to any one of claims 1 to 12 or a composition according to any one of claims 13 to 16 for use in reducing inflammation or controlling an allergic response in a mammal.

18. The process for the preparation of a compound of any one of claims 1 to 12 of the formula:

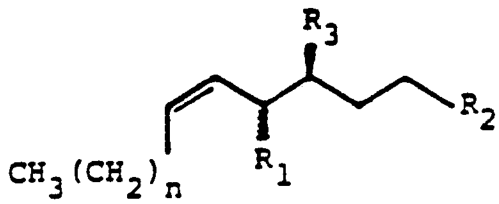

wherein $R_1$, $R_2$, $R_3$ and n are as defined in claim 1, which comprises reacting a compound of the formula:

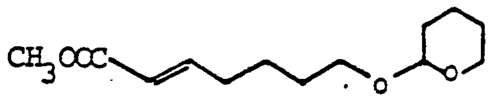

with a reducing agent to form an allylic alcohol of the formula:

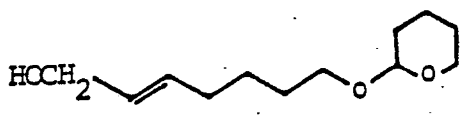

reacting the allylic alcohol with a first oxidizing agent to form the trans epoxy alcohol of the formula:

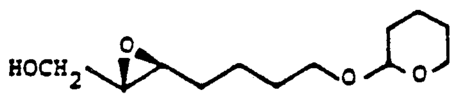

reacting the epoxy alcohol with a second oxidizing agent [dipyridine chromium (VI) oxide] to form an epoxy aldehyde of the formula:

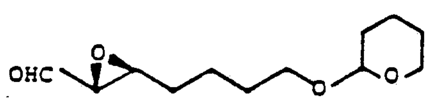

treating the epoxy aldehyde with a Wittig reagent of the formula:

$$CH_3(CH_2)_nCH_2P^{\oplus}PH_3Br^{\ominus}$$

to form an ethylenic epoxide of the formula:

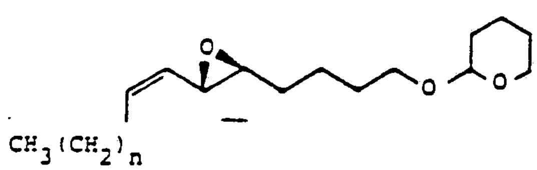

reacting the epoxide with a thiol of the formula:

$R_1SH$

to form a thio derivative of the formula:

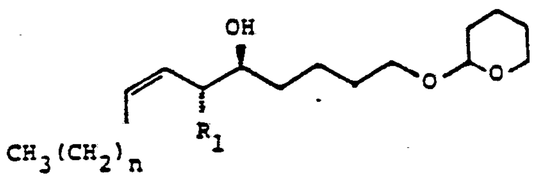

and reacting the product formed with an acid.

19. The process of claim 18 wherein the reducing agent is diisobutylaluminum hydride.

20. The process of claim 18 or claim 19 wherein the first oxidizing agent is the Sharpless Reagent and the second oxidizing agent in Pyridine $CrO_3$ complex.

21. The process of any one of claims 18 to 20 wherein the Witting reagent is dodecyl triphenylphosphonium bromide.

22. The process for the preparation of a compound of claim 1 of the formula:

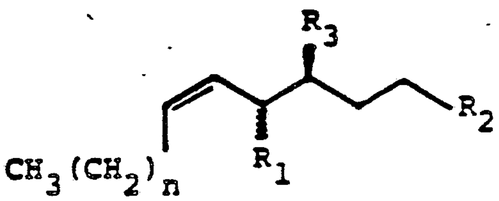

wherein $R_2$ is $-CH_2COOH$ or $-CH_2COO^-$; $R_3$ is hydroxy and $R_1$ is selected from the group consisting of thiophenyl, substituted thiophenyl, phenylsulfonyl, substituted phenylsulfonyl, cysteinyl, glutathionyl, cysteinylglycinyl, thioalkylamino, substituted thioalkylamino, sulfonylalkylamino, substituted sulfonylalkylamino, thioalkylacetamido, substituted thioacetamido, sulfonylalkylacetamido and substituted sulfonylalkylacetamido;

which comprises reacting a compound of the formula:

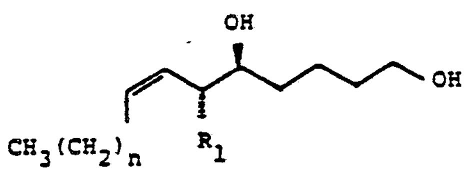

with silver carbonate to form a lactone of the formula:

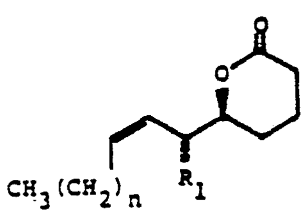

reacting the lactone with base to form the corresponding salt and treating the salt with acid to form the free acid.

23. The process of claim 22 wherein the base is sodium hydroxide and the acid is hydrochloric acid.

24. The process for the preparation of a compound of claim 1 of the formula:

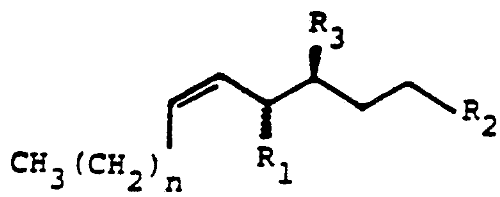

wherein $R_1$ is selected from the group consisting of thiophenyl, substituted thiophenyl, phenylsulfonyl, substituted phenylsulfonyl, cysteinyl, glutathionyl, cysteinylglycinyl, thioalkylamino, substituted thioalkylamino, sulfonylalkylamino, substituted sulfonylalkylamino, thioalkylacetamido, substituted thioacetamido, sulfonylalkylacetamido and substituted sulfonylalkylacetamido; $R_3$ is oximino and $R_2$ is tetrahydropyranyloxy and lower alkyl hydroxy;

which comprises reacting a compound of the formula:

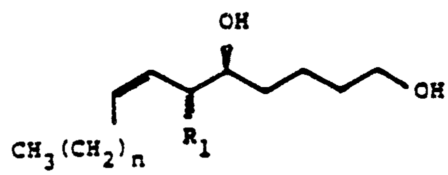

with an oxidizing agent to form a ketone of the formula:

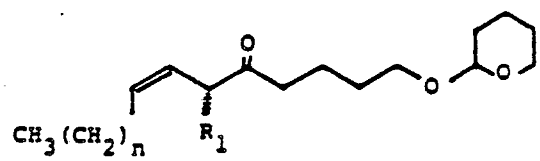

reacting the ketone with hydroxylamine hydrochloride to form an oxime of the formula:

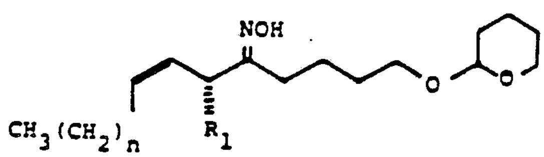

and treating the product formed with acid.

25. The process of claim 24 wherein the acid is p -toluenesulfonic acid.

26. The process of claim 24 or claim 25 wherein the oxidizing agent is oxalyl chloride/DMSO.

27. A method of producing a composition according to any one of claims 13 to 16 which comprises dispersing a compound of any one of claims 1 to 12 in a pharmaceutically acceptable carrier.